# EUROPEAN PATENT SPECIFICATION

(11) **EP 1 250 137 B1**
(45) Date of publication and mention of the grant of the patent: **15.08.2007**
(21) Application number: 01942563.6
(22) Date of filing: 22.01.2001
(51) Int. Cl.: A61K 31/00, A61K 31/505, A61K 31/55, A61P 19/02

(54) **JAK/STAT PATHWAY INHIBITORS AND THE USE THEREOF FOR THE TREATMENT OF PRIMARY GENERALIZED OSTEOARTHRITIS**
INHIBITOREN DES JAK/STAT-WEGES UND DEREN VERWENDUNG ZUR BEHANDLUNG VON ALLGEMEINER PRIMÄRER OSTEOARTHRITIS
INHIBITEURS DU TRAJET DE JAK/STAT ET LEUR UTILISATION POUR LE TRAITEMENT DE L'OSTÉOARTHRITE PRIMAIRE GÉNÉRALISÉE

(30) Priority: 24.01.2000 US 177872 P; 28.11.2000 US 723490
(43) Date of publication of application: 23.10.2002
(60) Divisional application: 06077137.5
(73) Proprietor: GENZYME CORPORATION, Cambridge, MA 02142 (US)
(72) Inventor: VASIOS, George, Brookline, MA 02445 (US)
(74) Representative: Adams, Harvey Vaughan John
(86) International application number: PCT/US2001/002033
(87) International publication number: WO 2001/052892

(56) References cited:
- EP-A- 0 711 773
- WO-A-01/41768
- WO-A-93/16703
- WO-A-94/24136
- WO-A-95/14464
- WO-A-97/11692
- WO-A-97/17360
- WO-A-99/15157
- WO-A-99/65495
- WO-A-99/65909
- US-A- 5 310 759
- US-A- 5 591 740
- US-A- 6 159 694
- UCKUN F M ET AL: "Pharmacokinetics and in vivo toxicity of Janus kinase 3 (JAK3) inhibitor WHI-P131 (4-(4'hydroxyphenyl)-amino-6,7-Dimethoxyqu inazoline)." BLOOD, vol. 94, no. 10 SUPPL. 1 PART 2, 15 November 1999 (1999-11-15), page 197b XP001002122 Forty-first Annual Meeting of the American Society of Hematology;New Orleans, Louisiana, USA; December 3-7, 1999 ISSN: 0006-4971
- GENG YU ET AL: "Cyclic GMP and cGMP-binding phosphodiesterase are required for interleukin-1-induced nitric oxide synthesis in human articular chondrocytes." JOURNAL OF BIOLOGICAL CHEMISTRY, vol. 273, no. 42, 16 October 1998 (1998-10-16), pages 27484-27491, XP001004498 ISSN: 0021-9258
- SALVATORI R ET AL: "PROSTAGLANDIN E-1 INHIBITS COLLAGENASE GENE EXPRESSION IN RABBIT SYNOVIOCYTES AND HUMAN FIBROBLASTS" ENDOCRINOLOGY, vol. 131, no. 1, 1992, pages 21-28, XP001004537 ISSN: 0013-7227
- KOLENKO VLADIMIR ET AL: "Downregulation of JAK3 protein levels in T lymphocytes by prostaglandin E2 and other cyclic adenosine monophosphate-elevating agents: Impact on interleukin-2 receptor signaling pathway." BLOOD, vol. 93, no. 7, 1 April 1999 (1999-04-01), pages 2308-2318, XP001002121 ISSN: 0006-4971
- KIRKEN ROBERT A ET AL: "Tyrphostin AG-490 inhibits cytokine-mediated JAK3/STAT5a/b signal transduction and cellular proliferation of antigen-activated human T cells." JOURNAL OF LEUKOCYTE BIOLOGY, vol. 65, no. 6, June 1999 (1999-06), pages 891-899, XP001004574 ISSN: 0741-5400
- BADGER ALISON M ET AL: "Inhibition of interleukin-1-induced proteoglycan degradation and nitric oxide production in bovine articular cartilage/chondrocyte cultures by the natural product, hymenialdisine." JOURNAL OF PHARMACOLOGY AND EXPERIMENTAL THERAPEUTICS, vol. 290, no. 2, August 1999 (1999-08), pages 587-593, XP000893197 ISSN: 0022-3565
- HUIBREGTSE BARBARA ET AL: "Z-debromohymenialdisine shows disease-modifying activity in an animal model of osteoarthritis." ARTHRITIS & RHEUMATISM, vol. 42, no. 9 SUPPL., 1999, page S254 XP001004668 63rd Annual Scientific Meeting of the American College of Rheumatology and the 34th Annual Scientific Meeting of the Association of Rheumatology Health Professionals;Boston, Massachusetts, USA; November 13-17, 1999 ISSN: 0004-3591
- EDER, CLAUDIA ET AL: "New Alkaloids from the Indopacific Sponge Stylissa carteri" J. NAT. PROD. (1999), 62(1), 184-187 , XP001023441
- PETTIT G R ET AL: "ANTINEOPLASTIC AGENTS. 168. ISOLATION AND STRUCTURE OF AXINOHYDANTOIN" CANADIAN JOURNAL OF CHEMISTRY, NATIONAL RESEARCH COUNCIL. OTTAWA, CA, vol. 68, no. 9, 1990, pages 1621-1624, XP000891506 ISSN: 0008-4042
- SUPRIYONO A ET AL: "BIOACTIVE ALKALOIDS FROM THE TROPICAL MARINE SPONGE AXINELLA CARTERI" ZEITSCHRIFT FUER NATURFORSCHUNG. SECTION C. BIOSCIENCES, XX, XX, vol. 50, no. 9-10, 1995, pages 669-674, XP000893152 ISSN: 0341-0382
- BRETON J J ET AL: "THE NATURAL PRODUCT HYMENIALDISINE INHIBITS INTERLEUKIN-8 PRODUCTION IN U937 CELLS BY INHIBITION OF NUCLEAR FACTOR-KAPPA B" JOURNAL OF PHARMACOLOGY AND EXPERIMENTAL THERAPEUTICS, AMERICAN SOCIETY FOR PHARMACOLOGY AND, US, vol. 282, no. 1, 1997, pages 459-466, XP002910635 ISSN: 0022-3565
- SUDBECK E A ET AL: "RECENT ADVANCES IN JAK3 KINASE INHIBITORS" IDRUGS, CURRENT DRUGS LTD, GB, vol. 2, no. 10, 1999, pages 1026-1030, XP000957840 ISSN: 1369-7056
- BEDNAR M. S. ET AL.: "Cyclic AMP-reguling agents inhibit endotoxin-mediated cartilage degradation" BIOCHEM. J., vol. 244, 1987, pages 63-68,
- LAMB, P. ET AL.: "Biochemical approaches to discovering modulators of the JAK/STAT pathway" DDT, vol. 3, no. 3, 1998, pages 122-130,
- LOUGHLIN J.: "The genetic epidemiology of human primary osteoarthritis: current status" EXPERT REVIEWS IN MOLECULAR MEDICINE, vol. 7, no. 9, 2005, pages 1-12,

## Description

### Cross-reference to Related Application

This application claims priority to US provisional application no. 60/177,872, filed January 24, 2000.

### Field of the Invention

The present invention is in the fields of molecular biology and orthopedics. The present invention is directed to novel treatments of JAK/STAT-mediated diseases or disorders, particutarly the JAK3-mediated disease or disorder primary generalized osteoarthritis using JAK3 inhibitors.

### Background of the Invention

### The Biology of Degenerative Joint Disease

Articular cartilage covers the ends of long bones within synovial joints to protect the underlying bone against normal shearing and compression forces that accompany body support and movement. Cartilage is composed of an extracellular matrix collagen. Contained within the collagen matrix are chondrocytes (i.e., specialized cartilage cells) and the ground substance. The ground substance is composed of proteoglycans and water. Collagen forms the matrix that imparts tensile strength, while proteoglycans form large aggregates that provide resistance to compression (Stockwell, 1991). Proteoglycans are large, strongly negative, hydrophilic molecules, which draw water. Under the normal pressure of joint function, water is expressed from the cartilage to lubricate the joint surface. Once pressure is relieved, the water is taken up by the proteoglycans of the cartilage. Water movement also provides the transport of nutrients and waste products to and from the chondrocytes: there is no blood supply to cartilage tissue. Maintaining cartilage integrity is critically important. Abnormal loading, either increasing or decreasing, affects the physical integrity of cartilage, influences cell metabolism, and induces biochemical changes, all of which can lead to cartilage degradation and the development of a degenerative joint disease, such as osteoarthritis (OA) (Mow et al., 1992).

Degenerative joint disease such as OA occurs widely in vertebrates. It is characterized as a progressive, irreversible, disease (Mankin and Brandt, 1991) resulting from cartilage degradation. Although the name osteoarthritis is suggestive of an inflammatory disease or disorder, OA arises from biochemical processes within cartilage that lead to cartilage degradation. In this regard, OA is distinguished from rheumatoid arthritis (RA) and seronegativespondyloarthropathies. Unlike RA, in which the synovium is the primary source of degradative enzymes, in OA, chondrocytes are the principal source of enzymes responsible for cartilage catabolism. Occasional and transient inflammatory responses, however, are often a separate, secondary complication associated with the progression of OA (Goldring, 1999).

Although the initiating event(s) in the development of OA is still unknown, the pathogenesis of OA likely involves an interaction of extrinsic mechanical factors and intrinsic cartilage metabolism. Ultimate causes of OA can range from physical trauma to the cartilage (secondary OA) to metabolic changes which affect normal cartilage maintenance processes, to low-grade intermittent inflammatory reactions, to genetic disorders, all of which may induce autolytic enzymes (Wilson, 1988, Goldring, 1999). Regardless of the initiating event or trauma that may trigger the onset of OA, the generally accepted model is that activated cytokines and/or receptors result in signal transduction to the chondrocyte nucleus, inducing gene expression of cartilage degrading enzymes and inflammatory agents.

Direct injury of cartilage can also cause injury to chondrocytes. Chondrocytes can respond to injury by producing degradative enzymes and by inducing inappropriate repair processes (Mow, et al., 1992). Biochemical changes in OA affect several cartilage components, including proteoglycan aggregates and collagens. Proteoglycan degradation products have been identified in the synovial fluid of OA patients (Lohmander et al., 1993). Decreased proteoglycan content in association with degraded collagen leads to the functional loss of normal matrix physiological properties.

Enzymatic breakdown of cartilage matrix is a key factor in degenerative joint disease onset and progression (Pelletier et al., 1992; Pelletier et al., 1993). Cartilage degrading enzymes known to play a major role in OA pathology include matrix metalloproteases (MMPs), aggrecanases, and serine and thiol proteases (Pelletier et al., 1997).

Matrix metalloproteases involved in OA include collagenases, stromelysins, and gelatinases. Collagenases are responsible for breakdown of the collagen type II scaffolding in cartilage. Collagenase-1 (MMP-1) and Collagenase-3 (MMP-13) have been identified in *in situ* OA cartilage. Levels of Collagenase-1 and -3 correlate with histological severity of OA-affected cartilage (Reboul et al., 1996). The stromelysins and gelatinases (including gelatinase-A and -B, also referred to as MMP-2 and MMP-9 respectively) are metalloproteoglycanases, also involved in OA. Stromelysin levels also correlate with histological severity of OA (Dean et al., 1989). In addition, stromelysin has also been implicated in the activation of procollagenase, thus amplifying its overall effect in OA pathology (Murphy et al., 1987). Neutrophil collagenase (also known as collagenase-2, or MMP-8) has been shown to cleave aggrecan at unique sites, including the site of aggrecanase cleavage, although not preferentially enough to be considered a true aggrecanase (Arner et al., 1997). These matrix metalloproteinases are believed to be primarily responsible for the damage to proteoglycan, collagen II, and collagen IX components of cartilage that occurs in OA (Dean, et al., 1989; Mort, et al., 1993; Buttle, et al., 1993).

Aggrecanase(s) represent a family of enzymes that degrade aggrecan, the major component of proteogylcan aggregates in cartilage. Aggrecanases are defined by their characteristic cleavage of aggrecan between Glu373-Ala374. Aggrecanases have been recently characterized as a sub-family of the disintegrin and metalloprotease (ADAM) family, containing multiple carboxy thrombospondin motifs that are responsible for extracellular matrix binding. These disintegrin and metalloprotease with thrombospondin motif (ADAMTS) compounds, specifically ADAMTS -4, -11 and possibly -1, possess characteristic cleavage-specific aggrecanase activity (Abbaszade et al., 1999; Tortorella et al., 1999).

Nitric oxide (NO), an inorganic free radical, has also been implicated in degenerative joint disorders. NO is enzymatically synthesized from 1-arginine by NO synthase (NOS). Two isoforms of NOS are currently known; constitutive NOS (cNOS) and inducible NOS (iNOS). Clinical measurement of nitrite and nitrate levels from the synovial fluid of OA patients indicates NO production in osteoarthritic joints (Farrell et al., 1992). In addition, NOS inhibitors have been reported to suppress some arthritic affects in rats (McCartney-Francis et al., 1993).

Cyclooxygenase 2 (COX 2) plays a major role in the synthesis of eicosanoids, locally-acting hormone-like molecules that function in a wide variety of biological processes relating to pain, fever, and inflammation. Specifically, COX 2 is required for the synthesis of prostaglandins, prostacyclins, and thromboxanes from arachidonic acid. NF-κB (a heterodimer of p65 and p50) is a transcription factor that activates the transcription of COX 2. Because COX 2 is required in the synthesis of prostaglandins, this enzyme and its transcription factor, NF-κβ, have also been targeted as key components in the onset and progression of OA.

The role of cytokines that regulate connective tissue metabolism and their concomitant intracellular signal transduction pathways have been extensively studied in regard to tissue degradation associated with joint diseases such as rheumatoid arthritis and osteoarthritis (see, e.g., Goldring, 1999; Pelletier et al., 1993). Research has revealed that inflammatory cytokines play a central role as biochemical signals that stimulate chondrocytes to release the various cartilage-degrading compounds mentioned above. Currently, the main cytokines believed to be associated with cartilage degradation are interleukin-1 and -6 (IL-1 and IL-6) and tumor necrosis factor-α (TNF-α).

IL-1 and TNF-α are reported to increase the synthesis (i.e., gene expression) of proteases including metalloproteases. Injections of 1L-1 and TNF-α in combination elicit greater cartilage degradation than either cytokine alone (Henderson and Pettipher, 1989; Page-Thomas, 1991). In addition, IL-1 has been reported to exhibit autocrine activity in chondrocytes (Attur et al., 1998; Pelletier ct al., 1993), producing a positive feedback mechanism. IL-1 and TNF-α also induce IL-6 expression in synovial fibroblasts, implicating IL-6 as an intermediate signal in the induction of other cellular (transcriptional) responses. IL-6 levels have been found to correlate with high levels of TNF-α, and are increased in the synovial fluids from OA tissue. IL-1 has been shown to play a major role in cartilage degradation observed in OA (Pelletier, et al., 1991; McDonnell, et al., 1992). It upregulates the synthesis and secretion of the metalloproteinases stromelysin and interstitial collagenase in a dose dependent manner (Stephenson, et al., 1987; Lefebvre, et al., 1990). Macrophage-like synovial cells are considered by some to be the major source of IL-1 and other cytokines that induce chondrocytes to express cartilage-degrading enzymes. Chondrocytes themselves are also known to produce IL-1 (Goldring, 1999).

Despite the extensive research efforts detailed above, manipulation of intracellular chondrocyte signal transduction pathway(s) in order to alter the course of mechanical and/or cytokine induction of cartilage degradation enzymes remains an elusive goal. Current research has focused upon the mitogen activated protein kinase (MAPK) family, which is not only activated by a diverse array of stimuli, but also regulates a number of transcription factors (especially activator protein-1, or AP-1) suspected to be responsible for the expression of MMP genes (e.g., collagenase-1 and -3) and certain inflammatory cytokines (Rutter et al., 1997; Pendas et al. 1997; and Lee et al., 1994). Stress-activated protein kinases (SAPKs, also referred to as JNKs), extracellular regulated kinases (ERKs), and p38 kinases have all been considered important proteins in the signal transduction pathway leading to the expression of cartilage degrading enzymes.

AP-1 is a heterologous protein complex that includes c-Jun and c-Fos polypeptides. AP-1 activation is also believed to play an important role in progressive bone and cartilage degenerative diseases (Firestein, 1996). AP-1 is believed to regulate the collagenase genes and stromelysin (Matrisian, 1994), and IL-1 is among the most potent inducers of collagenase and AP-1 in RA fibroblast-like synoviocytes (Zuoning et al. 1999).

### Degenerative Joint Disease Treatment

Current approaches to develop therapeutics for degenerative joint diseases such as OA include synthesis of inhibitors of cartilage degrading enzymes, regulation of cytokine/cell receptor levels, and regulation of protein kinases generally. For example, it has been reported that some protein kinase C inhibitors can be used to alleviate, *inter alia,* PKC-mediated inflammation generally (Nambi and Patil, 1993; Nambi and Patil, 1997). Certain of these inhibitors, including 4-(2-amino-4-oxo-2-imidazolin-5-ylidene)-2-bromo-4,5,6,7-tetrahydropyrrolo (2,3-c) azepine-8-one (hymenialdisine; hereinafter "H"), and 4-(2-amino-4-oxo-2-imidazolin-5-ylidene)-4,5,6,7-tetrahydropyrrolo(2,3-c) azepine-8-one (debromohymenialdisine; hereinafter "DBH") and various physiologically active salts thereof, were later found to inhibit the IL-1 induced degradation of glycosaminoglycan and extracellular matrix by chondrocytes in culture and in explants of articular cartilage (Chipman and Faulkner, 1997).

Recently, the tyrosine kinase inhibitors genistein, herbimycin A, 4,5-dianilinophthalimide (DAPH), tyrphostin AG 82 and tyrphostin AG 556 also have been found to reduce or prevent cartilage degradation by chondrocytes in vitro (Sharpe et al., 1997). WO 97 11692 discloses that protein tyrosine kinase inhibitors, such as tyrphostins, are effective for treating osteoarthritis. US 5310759 discloses the use of a cyclic adenosine monophosphate agonist or inducer, such as 3-isobutyl-1-methylxanthine (IBMX) or forskolin, for the treatment of degenerative joint diseases, such as osteoarthritis.

Nevertheless, many of the cellular mechanisms that specifically regulate expression of cartilage degrading enzymes involved in degenerative joint disease remain unknown. Discovery of a specific signal transduction pathway that regulates cartilage degrading enzymes, either directly or through activating intermediates, would represent a tremendous advance in the understanding of the induction and progression of degenerative joint disease and provide new avenues for the development of a new class of effective therapeutics for the treatment of such diseases.

### The Janus Kinase (JAK) pathway

A separate and distinct signal transduction pathway, never before associated in any respect with the regulation of cartilage degrading enzymes, nor with the IL-1 signal transduction pathway, is the JAK/STAT pathway. A wide variety of polypeptide cytokines, lymphokines, and growth factors activate (via cytokine receptors) the JAK family (reviewed by Aringer et al., 1999). Receptor-activated JAK associations proceed to activate (i.e., tyrosine phosphorylate) STAT (Signal Transducers and Activators of Transcription) proteins. JAKs are believed to be to be the principal activators of the five currently known STAT proteins (Silvennoinen et al., 1997).

The current model for STAT activation is that JAKs phosphorylate specific tyrosine residues within the activated cell receptor, creating docking sites for STATs to bind at their Src homology 2 (SH2) domains. JAKs catalyze STAT phosphorylation, activating STAT dimerization and disengaging the STATs from the receptor. STAT dimers then translocate to the cell nucleus, where they function as transcription factors, binding to, for example, interferon DNA promoter regions (IRE and GAS) (Darnell Jr. et al., 1994; Ihle, 1995; Ihle, 1994; Darnell, 1997).

Further upstream, JAK activation is directly linked to cellular cytokine transmembrane receptors that lack intrinsic kinase activity. JAKs are capable of binding to the cytoplasmic motifs of these receptors. The cellular receptors act to recruit/activate JAKs as their nonreceptor protein kinase, to direct intracellular signaling (Aringer et al., 1999). One model proposes that ligand-induced receptor dimerization or oligomerization brings about the local aggregation of JAK molecules and results in JAK activation by a cross-phosphorylation mechanism (Taniguchi, 1995).

The JAK family members, currently consisting of JAK1, JAK2. TYK2, and JAK3, are nonreceptor tyrosine kinases. JAK proteins contain a highly conserved catalytic domain, found in other tyrosine kinases (Firmbach-Kraft, 1990; Hanks et al., 1991; Hunter, 1991; Wilks, 1989). Unlike most other tyrosine kinases, however, JAKs are localized in the cytoplasm and contain a second kinase-like domain of unknown function, but do not contain SH2 or SH3 domains, signal peptide sequences, or transmembrane domains, (Harpur et al., 1992; Wilks et al., 1991).

JAK proteins are known to be involved in signaling from a number of cytokines that act on hemopoietic cells. JAK signal transduction is activated by: IFN-α, -β, and -γ (interferons); IL-2,-3, - 4, -6, -7, -17 (interleukins); GM-CSF (granulocyte macrophages colony stimulating factor); EPO (erythropoietin); GH (growth hormone); CNTF (ciliary neurotrophic factor); LIF (leukemia inhibitory factor); OSM (oncostatin M); and PRL (prolactin) (Argetsinger, 1993; Gauzzi et al., 1996; Helden and Purton (eds.) 1996; Ihle, 1996; Liu et al., 1997; Luttichen, 1994; Muller et al., 1993; Schindler and Darnell Jr., 1995; Stahl et al., 1994; Subramaniam et al., 1999; Velazquez et al., 1992; Watling et al., 1993; Witthuhn et al., 1993; Rui et al., 1994).

Absolute correlation has been demonstrated between JAK activation and activation of several cytokine-induced downstream signaling events. These include; phosphoinositol 3-kinase (PI-3K), Shc, insulin receptor substrate -1 and -2 (IRS -1 and -2), as well as Vav phosphorylation and induction of c-Myc, c-Fos, c-Jun, Pim, and CIS genes (Silvennoinen et al., 1997).

JAK/STAT proteins have been found to be present and modulated *in vivo* in embryonic and postnatal brain, indicating a role during brain development (De-Fraja et al., 1998). The JAK/STAT pathway has been found to be important in brain tumors (e.g., meningiomas). And, the JAK/STAT pathway has also been found to serve as a signal transduction pathway in the pathogenesis of diabetic nephropathy.

### Janus Kinase 3 (JAK3)

JAK3 is the newest member of the JAK protein tyrosine kinase family. Three splice variants of JAK3 have been reported in hematopoietic and epithelial cancer cells. The JAK3 splice variants contain identical amino-terminal regions but diverge at the C-terminus (Lai et al., 1995; Gurniak and Berg, 1996). The functional significance of these splice variants is not completely understood. The amino terminal JH 7-6 domains (amino acids 1-192) of JAK3 have been shown to be necessary and sufficient for its interaction with the IL-2R subunit yc (Chen et al., 1997).

The JAK/STAT signal transduction pathway, and in particular the role of JAK3, represents a significant point of therapeutic intervention for various diseases and disorders. The full extent and function of the JAK/STAT pathway remains unclear, however. There is a need in the art to understand the JAK/STAT pathway more completely, and methods for regulating this signal transduction pathway will provide important new therapeutics in the treatment of diseases or disorders mediated by JAK/STAT.

### Summary of the Invention

The present invention is based upon the discovery that the JAK/STAT signal transduction pathway, and specifically JAK3, is involved in the initiation and progression of degenerative joint disease. Specifically, JAK3 inhibitors have been shown to block IL-1 induced expression of genes known to be involved in the development and progression of OA. Also demonstrated herein for the first time is that JAK3 is expressed in chondrocytes in at least two forms. Evidence is presented demonstrating that molecules such as DBH and H, which have been shown effective in treating cartilage degradative disorders such as OA in animal models, operate as JAK3-specific inhibitors which reduce or suppress, directly and/or indirectly, the expression of various cartilage degrading and inflammatory-mediating factors. Finally, evidence is provided that demonstrates that other JAK3 inhibitors can also effectively suppress, directly and/or indirectly, the expression of various cartilage degrading factors in a manner identical to that of DBH and H. The identification of this novel role of the JAK/STAT pathway in the degeneration of cartilage and the identification of and determination of the specific function of JAK3 inhibitors in preventing the pathology of such diseases provide new avenues for the development of a class of therapeutics effective for the treatment of degenerative joint disease.

The present invention is directed to the use of a JAK-3 inhibitor according to claim 1 to treat a disease or disorder involving cartilage degradation, which is primary generalized osteoarthritis.

The JAK/STAT inhibitors may regulate expression of a cartilage degrading enzyme, or of pro-inflammatory agents in a chondrocyte including iNOS, COX-2 or NF-κB or of a proinflammatory cytokine in a chondrocyte, including IL-6, TNF-α and IL-1.

Assays involving JAK3 interactions, such as an assay for detecting compounds useful for treating a disease or disorder involving cartilage degradation by detecting compounds capable of inhibiting JAK3, and those compounds discovered using this assay, are disclosed.

### Brief Description of the Drawings

**FIG. 1** provides a comparison of partial JAK3 cDNA sequence obtained from RT-PCR analysis of cultured human chondrocytes to published human JAK3 cDNA (GenBank Accession #U09607).
**FIG. 2** provides a comparison of partial JAK3 cDNA sequence obtained from RT-PCR analysis of mRNA taken directly from an osteoarthritic cartilage sample.
**FIG. 3** provides a comparison of published human JAK3 cDNA (GenBank Accession #U09607)and JAK3 cDNA isolated from a human chondrocyte cDNA library.
**FIG. 4** illustrates the results of northern blot analysis of JAK3 mRNA in normal human articular chondrocytes.

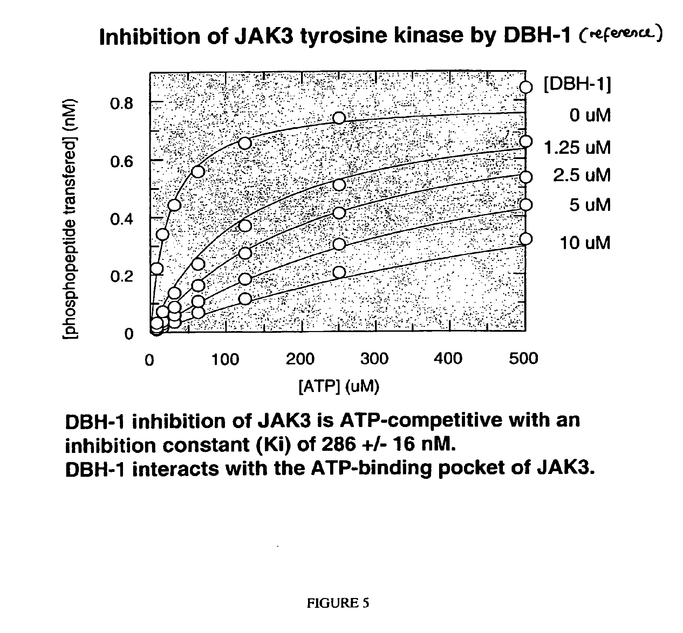

**FIG. 5** illustrates DBH inhibition of JAK3 determined by ELISA analysis. (reference)
**FIG. 6** provides an illustrative comparison of JAK3 inhibition by H (979) and DBH (5025) determined by ELISA analysis. (reference)
**FIG. 7** provides an illustrative comparison of substrate inhibition by different variant forms of DBH determined by ELISA analysis. **FIG. 7A** depicts the inhibition curves of two salt forms (DBH-2S and DBH-1) and one free base form (DBH-2FB) of DBH. **FIG. 7B** illustrates comparative inhibition of free base DBH to ZAP-70, LCK, BTK, IGF, and JAK3. (reference)
**FIG. 8 (A&B)** illustrate the results of northern blot analysis of DBH inhibition of mRNAs of various cartilage degrading components in human articular chondrocytes. (reference)
**FIG. 9** graphically depicts inhibition of IL-1 induced aggrecanase activity by different variant forms of DBH. (reference)
**FIG.10** illustrates the results of northern blot analysis of JAK3-specific inhibitor, 4-(4'-hydroxyphenyl)-amino-6,7-dimethoxyquinazoline (known in the art as WHI-P131, and identified in the figure as J1030) inhibition of mRNA of various cartilage degrading components in human articular chondrocytes.

### Detailed Description of the Invention

Disclosed herein is the first report describing a role for JAK/STAT signal transduction in chondrocytes and its involvement in the initiation and progression of degenerative joint disease. Specifically, the inventor has discovered that DBH and H, currently known to be effective therapeutics in animal models of degenerative joint disease, such as OA, in fact operate by disrupting JAK/STAT signal transduction in chondrocytes, specifically by acting as JAK3 inhibitors. To confirm this novel role of the JAK/STAT pathway in degenerative joint disease, evidence is presented demonstrating that an additional known JAK3 inhibitor, previously unknown to effect regulation of cartilage degradative factors, also regulates those cellular processes in a manner identical to DBH and H.

As a result of these discoveries, a novel class of compounds, i.e., JAK/STAT inhibitors, and preferably JAK3 inhibitors other than DBH and H, are identified as being useful for altering the course of diseases involving cartilage degeneration. Thus, in one embodiment, the present invention is directed to the use of a compound which inhibits JAK-3, other than hymerialdisine (H) or debromohymerialdisine (DBH), in the manufacture of a pharmaceutical composition for inhibiting the progression or likelihood of developing.

A "JAK/STAT inhibitor" refers to any compound capable of downregulating or otherwise decreasing or suppressing the amount and/or activity of JAK-STAT interactions. JAK inhibitors downregulate the quantity or activity of JAK molecules. STAT inhibitors downregulate the quantity or activity of STAT molecules. Inhibition of these cellular components can be achieved by a variety of mechanisms known in the art, including, binding directly to JAK (e.g., a JAK-inhibitor compound binding complex, or substrate mimetic), binding directly to STAT, or inhibiting the expression of the gene, which encodes the cellular components. The JAK/STAT inhibitor of the present invention is a AK3 inhibitor other than hymerialdisine (H) or debromohymerialdisine (DBH). Generally, JAK/STAT inhibitors may be proteins, polypeptides, small molecules and other chemical moieties, or nucleic acids.

Mutants, variants, derivatives and analogues of the aforementioned inhibitors may also be useful. As used herein, "mutants, variants, derivatives and analogues" refer to molecules with similar shape or structure to the parent compound and that retain the ability to act as JAK 3 inhibitors. For example, any of the JAK 3 inhibitors disclosed herein may be crystalized, and useful analogues may be rationally designed based on the coordinates responsible for the shape of the active site(s). Alternatively, the ordinarily skilled artisan may, without undue experimentation, modify the functional groups of a known inhibitor and screen such modified molecules for increased activity, half-life, bio-availability or other desirable characteristics. Where the JAK 3 inhibitor is a polypeptide, fragments and modifications of the polypeptide may be produced to increase the ease of delivery, activity, half-life, etc. Again, given the level of skill in the art of synthetic and recombinant polypeptide production, such modifications may be achieved without undue experimentation.

Examples of JAK/STAT inhibitors which may be useful in the methods of this invention include. PIAS proteins, which bind and inhibit at the level of the STAT proteins (Chung et al., 1997); members of an SH2 containing family of proteins, which are able to bind to JAKs and/or receptors and block signaling (see, for example, Aman and Leonard, 1997; Nicholson and Hilton, 1998); cytokine-inducible Src homology 2-containing (CIS) protein, an inhibitor of STAT signaling (Yoshimura et al., 1995); CIS-related proteins, which can inhibit STAT signaling or directly bind to Janus kinases (Yoshimura et al., 1995; Matsumoto et al, 1997; Starr et al., 1997; Endo et al., 1997; Naka et al., 1997); Suppressor of Cytokine Signaling-I protein (SOCS-1, also referred to as JAB or SSI-1), which appears to associate with all JAKs to block the downstream activation of STAT3 (Ohya et al., 1997); Tyrphostins, which are derivatives of benzylidene malononitrile, resembling tyrosine and erbstatin moieties (Gazit et al., 1989); AG-490, a member of the tyrophostin family of tyrosine kinase inhibitors (Wang et al. 1999, also Kirken et al., 1999); 4.5-dimethoxy-2-nitrobenzoic acid and 4,5-dimethoxy-2-nitrobenzamide, which specifically inhibit JAK3 (Goodman et al. 1998); 4-(phenyl)-amino-6,7-dimethoxyquinazoline (parent compound WHI-258) and derivatives of this compound which are structurally-derived from dimethoxyquinazoline compounds (Sudbeck et al. 1999); compounds containing a 4'-OH group, including 4-(4'-hydroxyphenyl)-amino-6.7-dimethoxyquinazoline (WHI-P131). 4-(3'-bromo-4'-hydroxylphenyl)-amino-6,7-dimethoxyquinazoline (WHI-P154), and 4-(3',5'-dibromo-4'-hydroxylphenyl)-amino-6,7-dimethoxyquinazoline (WHI-P97); WHI-P180, another dimethoxyquinazoline compound (Chen et al., 1999); and cAMP elevating agents, such as forskolin, a direct activator of adenylate cyclase and dibutyryl CAMP, and 3-isobutyl-1-methylxanthine (IBMX), an inhibitor of cAMP phosphodiesterase (Kolenko et al.. 1999).

As used herein, "DBH" and "H" refer to debromohymenialdisine and hymenialdisine, respectively, as well as to various analogues and physiologically active salts thereof including, free base DBH and trifluouroacetic acid and methanesulfonic acid forms of DBH. Analogues of DBH and H include compounds which contain a five-membered, nitrogen-containing heterocyclic ring bonded to the four position of the pyrroloazepine ring found in DBH. Examples of analogues include hymenin and axinohydantoin. Specifically, analogues of DBH and H contain the structure:

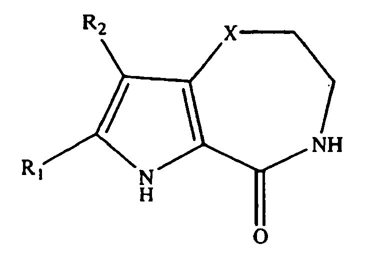

Wherein R₁ and R₂ are each independently selected from the group consisting of-H and a halogen and X is selected from the group consisting of: 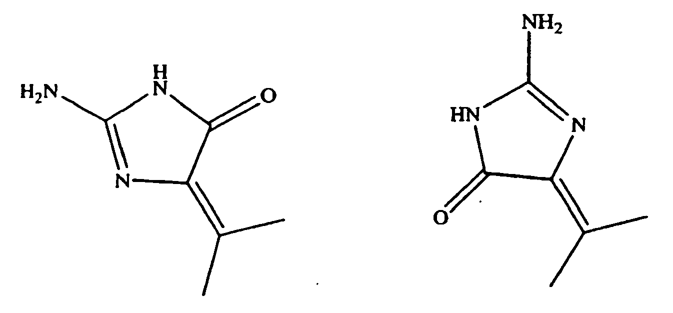 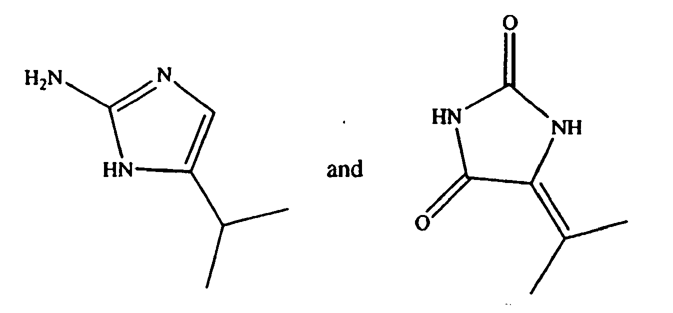 as set forth in Chipman and Faulkner (1997; wherein each of the above X radical group chemical structures include depiction of the chemical bond to the four position of the pyrroloazepine ring found in DBH).

As used herein, a "pharmaceutically effective amount" of a JAK/STAT inhibitor is an amount effective to achieve the desired physiological result, either in cells treated *in vitro* or in a subject treated *in vivo*. Specifically, a pharmaceutically effective amount is an amount sufficient to inhibit, for some period of time, one or more of the clinically defined pathological processes associated with the disease state at issue. The effective amount may vary depending on the specific JAK/STAT inhibitor selected, and is also dependent on a variety of factors and conditions related to the subject to be treated and the severity of the disorder. For example, if the inhibitor is to be administered *in vivo,* factors such as the age, weight and health of the patient as well as dose response curves and toxicity data obtained in preclinical animal work would be among those considered. If the inhibitor is to be contacted with the cells *in vitro,* one would also design a variety of pre-clinical *in vitro* studies to assess such parameters as uptake, half-life, dose, toxicity, etc. The determination of a pharmaceutically effective amount for a given agent is well within the ability of those skilled in the art.

JAK/STAT inhibitor other than DBH or H regulate expression of a cartilage degrading enzyme in a cell by contacting the cell with a pharmaceutically effective amount thereof. Cartilage degrading enzymes include matrix metalloproteases, aggrecanases and serine and thiol proteases. Preferred matrix metalloproteases include stromelysins (e.g., stromelysin-1), gelatinase A, gelatinase B, collagenase 1, collagenase 3, and neutrophil collagenase. Preferred aggrecanases include ADAMTS-1, ADAMTS-4, and ADAMTS-11. The cells amenable to such treatment include any cell that expresses such cartilage degrading enzymes, including chondrocytes and synoviocytes.

As used herein, the term "regulating expression" and/or activity generally refers to any process that functions to control or modulate the quantity or activity (functionality) of a cellular component. Static regulation maintains expression and/or activity at some given level. Upregulation refers to a relative increase in expression and/or activity. Downregulation is a relative decrease in expression and/or activity. Regulation is preferably the downregulation of a cellular component. As used herein, downregulation is synonymous with inhibition of a given cellular component.

JAK/STAT inhibitors other than DBH or H regulate expression of pro-inflammatory agents in a chondrocyte, including iNOS, COX-2 or NF-κB. by contacting the chondrocyte with a pharmaceutically effective amount thereof.

A pharmaceutically effective amount of a JAK/STAT inhibitor other than DBH or H regulates expression of a proinflammatory cytokine in a chondrocyte, including IL-6, TNF-α and IL-1.

Such a JAK/STAT inhibitor may regulate expression of cartilage degrading enzymes, pro-inflammatory agents and pro-inflammatory cytokines in synoviocytes.

Routes of administration of a JAK/STAT inhibitor according to claim 1 to a subject may include parenteral (including subcutaneous, intravenous, intramedullary, intraarticular, intramuscular, or intraperitoneal injection) rectal, topical, transdermal or oral. For example, the JAK/STAT inhibitor may be administered intraarticularly into a localized affected region (e.g., joint) of the subject, thus maximizing the therapeutic effect in that region, while minimizing effects to unaffected regions. The inhibitor may also be administered topically near the affected region. Alternatively, the inhibitor may be administered orally, for example, in capsules, suspensions or tablets.

The JAK/STAT inhibitor may be administered to a subject in a single dose or in repeat administrations and in any of a variety of physiologically acceptable salt forms, and/or with an acceptable pharmaceutical carrier as part of a pharmaceutical composition. Physiologically acceptable salt forms and standard pharmaceutical formulation techniques are well known to persons skilled in the art (see, for example, Remington's Pharmaceutical Sciences, Mack Publishing Co.).

Because the present disclosure teaches for the first time the functional role of the JAK/STAT pathway in chondrocytes in the onset and progression of cartilage degrading diseases or disorders, an assay for detecting novel compounds useful for treating such diseases or disorders, as well as those useful compounds, which have been identified by that assay are disclosed. The assays identify compounds useful for treating cartilage degrading diseases or disorders operate by screening a candidate compound, or library of candidate compounds, for its ability to inhibit JAK3 activity. A variety of assay protocols and detection techniques are well known in the art and easily adapted for this purpose by a skilled practitioner. Such assays include high throughput assays, *in vitro* and *in vivo* cellular and tissue assays.

Having now described the present invention in detail, the same will be more clearly understood by reference to the following examples, which are included for purposes of illustration only.

### EXAMPLE 1: Preparation of Human Articular Chondrocyte Cultures

To study the cellular pathology associated with degenerative cartilage diseases or disorders such as OA, human cartilage cell (human chondrocyte) cultures were prepared.

Normal human articular cartilage slices were obtained from the knee of a 30 year old Caucasian male within 48 hours of autopsy (National Disease Research Interchange (NDRI), Philadelphia, PA). Articular chondrocytes were released from minced slices of cartilage by enzymatic digestion (0.1% *Clostridium histolyticum* collagenase; Worthington, Freehold, NJ) in Dulbecco's modified Eagle medium (DMEM; GIBCO/BRL, Gaithersburg, MD) at 37°C overnight. Incompletely digested material was digested an additional three hours, using 0.25% collagenase and 0.05% trypsin in DMEM.

Chondrocytes were seeded on plastic in monolayer, and cultured to confluence in the presence of DMEM, 10% fetal bovine serum (FBS; HyClone, Logan, UT), 100 U/ml penicillin (pen), and 100 *µ*g/ml streptomycin (strep), at 37°C, 8% CO₂, with media changes every third day. At confluence, first passage chondrocytes were harvested by trypsinization, labeled as cell strain HC30-0198, and stored in liquid nitrogen in the presence of DMEM, pen/strep, 40% FBS, and 10% dimethyl sulfoxide for future use.

### EXAMPLE 2: JAK3 Expression in Normal Human Articular Chondrocytes

To demonstrate JAK3 expression in human chondrocytes, reverse transcription polymerase chain reaction (RT-PCR) using JAK3 specific primers was performed on total RNA isolated from normal adult human chondrocytes cultured in monolayer.

Normal adult human articular chondrocytes were isolated from cartilage slices and cultured as taught in Example 1 above. Frozen chondrocyte cell strain HC30-0198 was thawed, cultured in monolayer (second passage) as described above, and cultured to 5 days post confluence.

Total RNA was extracted from the 5-day post confluent human articular chondrocytes using the Qiagen Rneasy® Kit (QIAGEN, Valencia, CA), according to the manufacturer's suggested protocol.

JAK3 primers were designed from the published human JAK3 gene and cDNA sequences (GenBank Accession Nos. U70065 and U09607, respectively). Three JAK3-gene-specific DNA primers targeted exons 18 and 19 of the human JAK3 gene (Table I).

**Table 1. Human JAK3 DNA Primers.**

| Primer | Region/Strand | Sequence |
|---|---|---|
| JK3-1 | exon 18 (sense) | 5' GGT CAT GGA GTA CCT GCC 3' |
| JK3-2 | exon 19 (antisense) | 5' GTT GTC CGA GAG GGA TTC GG 3' |
| JK3-3 | exon 19 (antisense) | 5' GCG GAC CAC GTA GTA GTC 3 |

First-strand cDNA was made using the J-K3-2 primer, total RNA, and the Ready-To-Go^{®} You-Prime First-Strand Beads and method (Amersham Pharmacia Biotech, Piscataway, NJ).

The cDNA product (8 *µ*l) was amplified in a 100 *µ*l reaction using: 10 *µ*l of PCR 10X buffer, 2.4 *µ*l of 25 mM MgCl₂0.5 *µ*l of Taq polymerase using a hot start (Boehringer Mannheim, Indianapolis, IN), 73 *µ*l of water, and 3 *µ*l each of 10 *µ*M JK3-1 and JK3-3 primers. The hot start PCR protocol consisted of an initial denaturation for 5 min at 94°C (followed by the addition of Taq polymerase), followed by: denaturation for 30 sec at 94°C; annealization for 30 sec at 56°C; extension for 30 sec at 72°C. Denaturation, annealization, and extension proceeded for 40 cycles, followed by a final extension for 5 min at 72°C.

RT-PCR-generated a 254 base pair DNA product, which was purified by electrophoresis in a 2% SeaKem^{®} GTG^{®} agarose gel (FMC BioProducts, Rockland, ME) using the QIAquick™ gel extraction kit (QIAGEN, Valencia, CA).

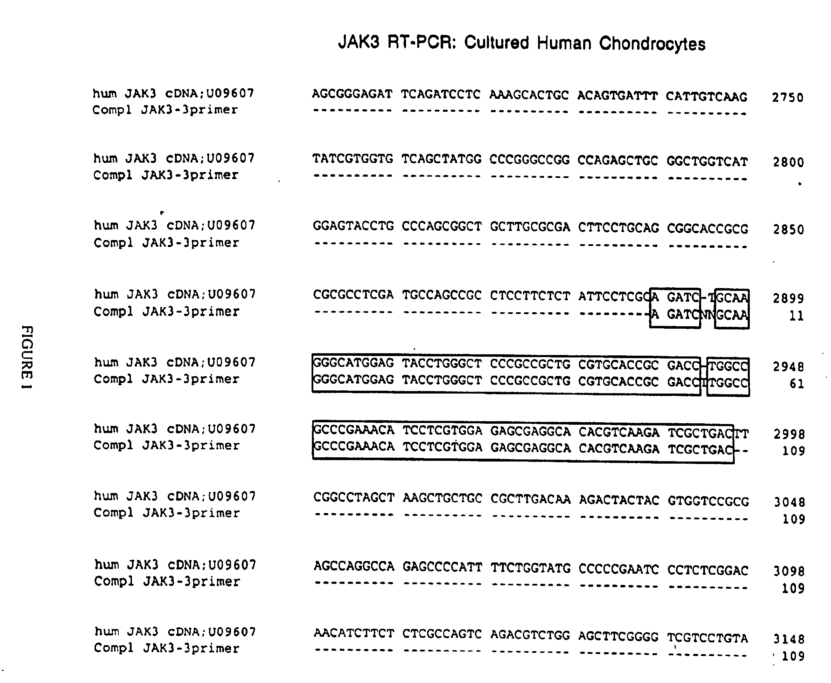

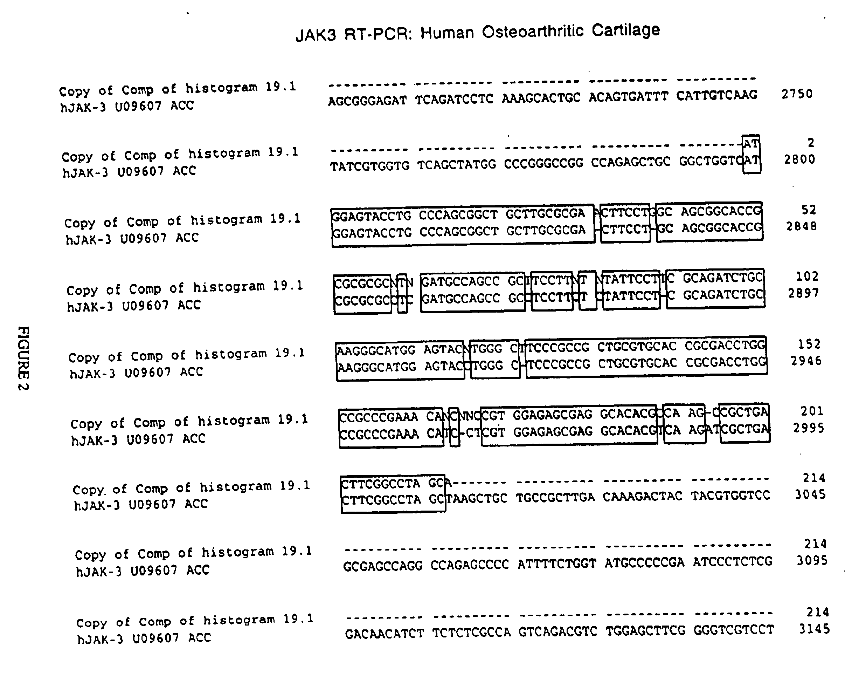

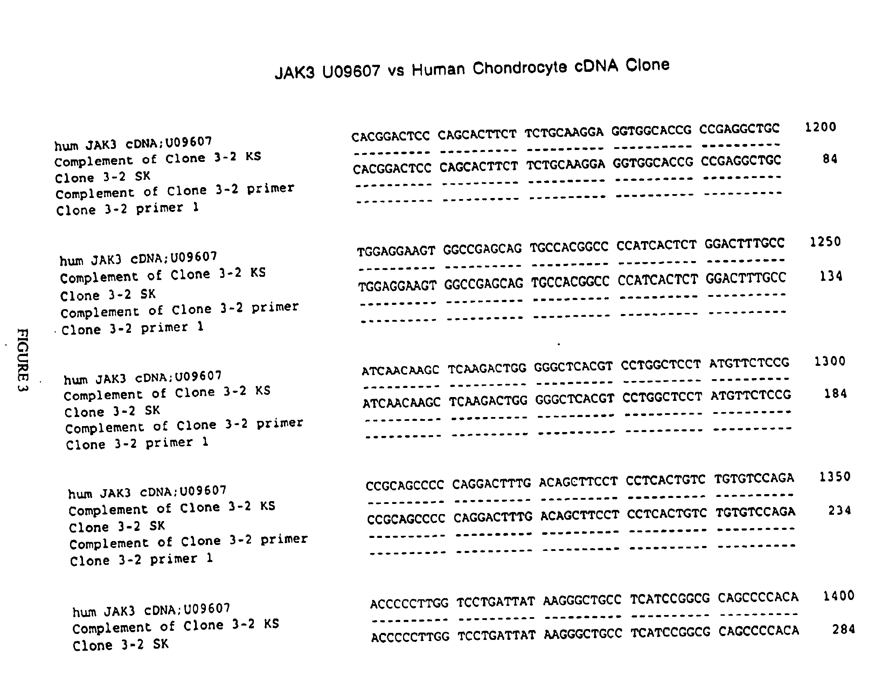

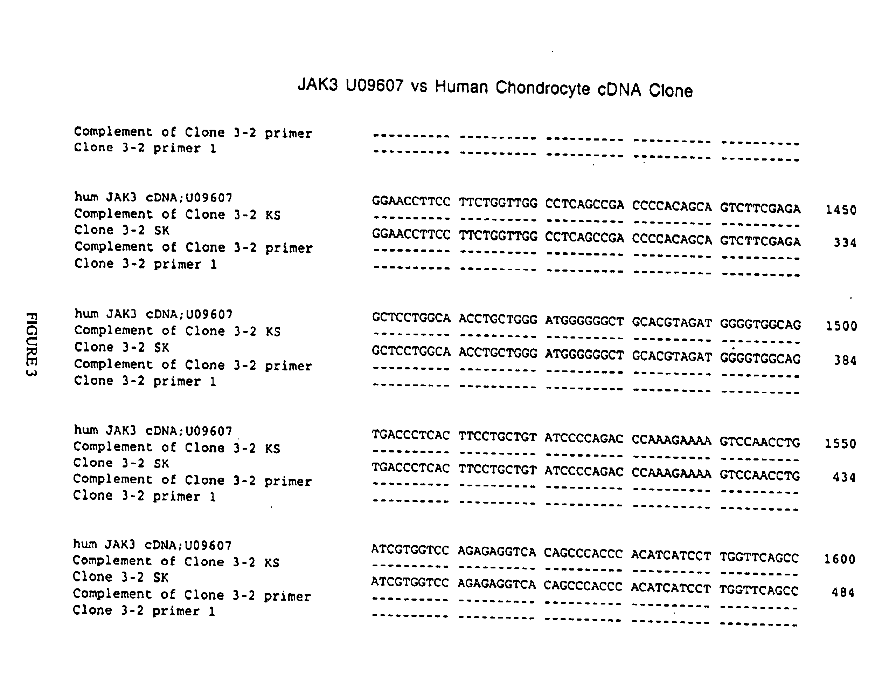

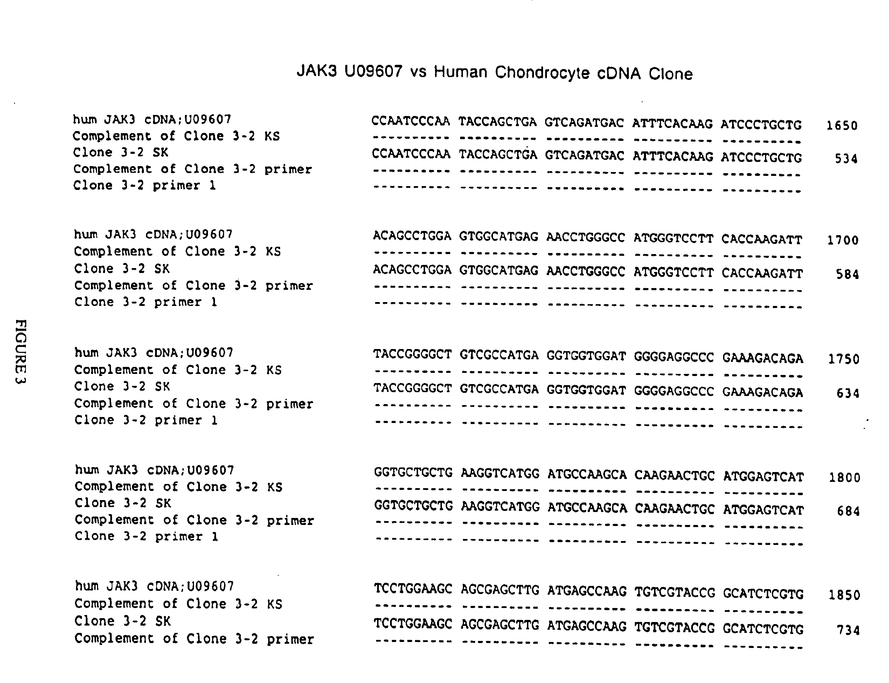

The RT-PCR product was sequenced using the JK3-3 primer and the AmpliCycle^{®} Sequencing reagents and protocol (Perkin Elmer, Foster City, CA). A 109 nucleotide DNA sequence was obtained from the cycle sequencing reaction of one strand of the RT-PCR product, with 106 nucleotides showing 100% identity to the GenBank human JAK3 cDNA sequence (Accession No. U09607) (FIG. 1). The three nucleotide discrepancies were attributed to the suboptimal quality of the RT-PCR-generated sequencing template and DNA sequencing gel.

### EXAMPLE 3: JAK3 Expression in Articular Chondrocytes from Human Osteoarthritic Cartilage

To compare JAK3 expression in chondrocytes from human osteoarthritic cartilage to that found in normal human chondrocytes of Example 2 above, RT-PCR using JAK3 specific primers was performed on total RNA isolated directly from adult human osteoarthritic cartilage.

Slices of osteoarthritic articular cartilage from the right knee of a 47-year-old Caucasian female undergoing total knee replacement were obtained from the NDRI. The cartilage was snap-frozen into liquid nitrogen immediately upon harvesting. A total of 2-4 grams of intact cartilage were pulverized into a powder in the presence of liquid nitrogen using a SPEX mill (SPEX CertiPrep, Inc., Metuchen, NJ). Total RNA was isolated from the pulverized cartilage using the TRIspin method describe by Reno et al. (1997).

RT-PCR was performed on the total RNA sample, using primers JK-1, JK-2, and JK-3 as described in Example 2. The RT-PCR generated a 254 base-pair-DNA product, which was subsequently purified from a 2% SeaKem^{®} GTG^{®} agarose gel (FMC BioProducts, Rockland, ME) using the QlAquick gel extraction kit (QIAGEN, Valencia, CA).

The RT-PCR product was sequenced using the JK3-3 primer and the ABI Prism Dye Terminator Cycle Sequencing Ready Reaction Kit (PE Applied Biosystems, Foster City, CA) according to the manufacturer's suggested protocol. The DNA sequence was determined using an ABI PRISM^{®} 377 DNA Sequencer (PE Applied Biosystems, Foster City, CA). A 214 bp sequence was determined.

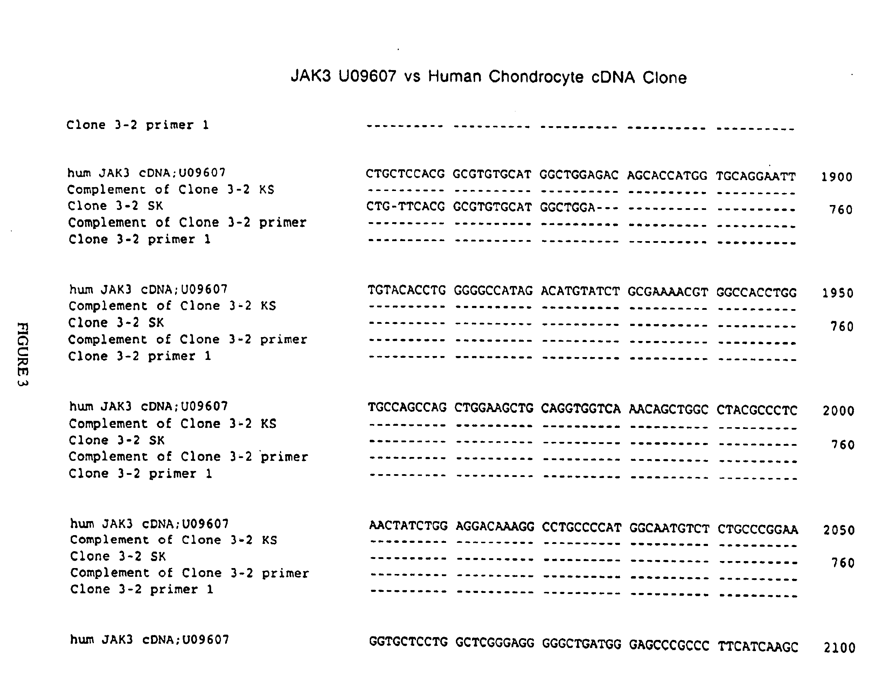

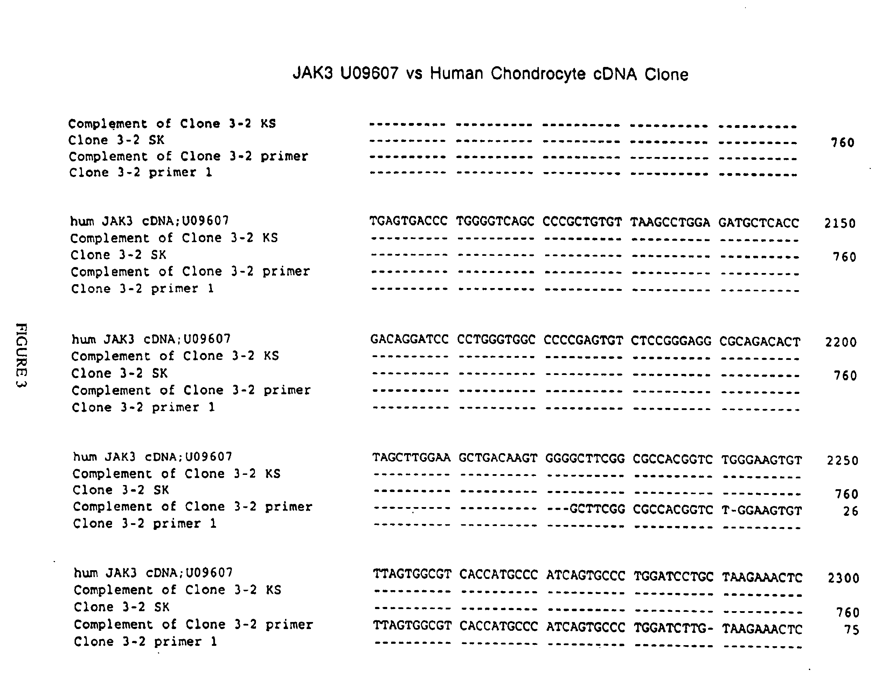

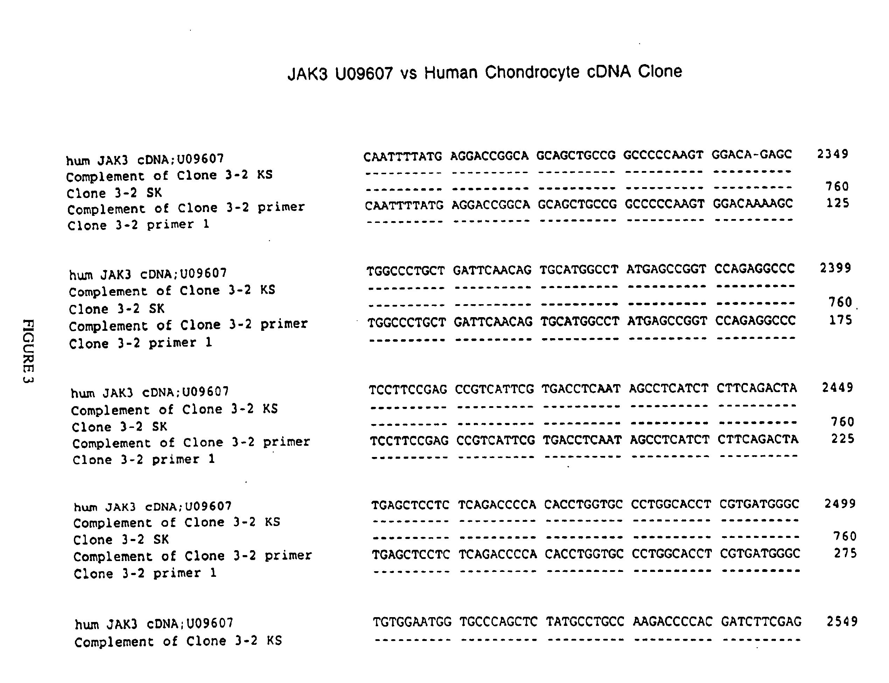

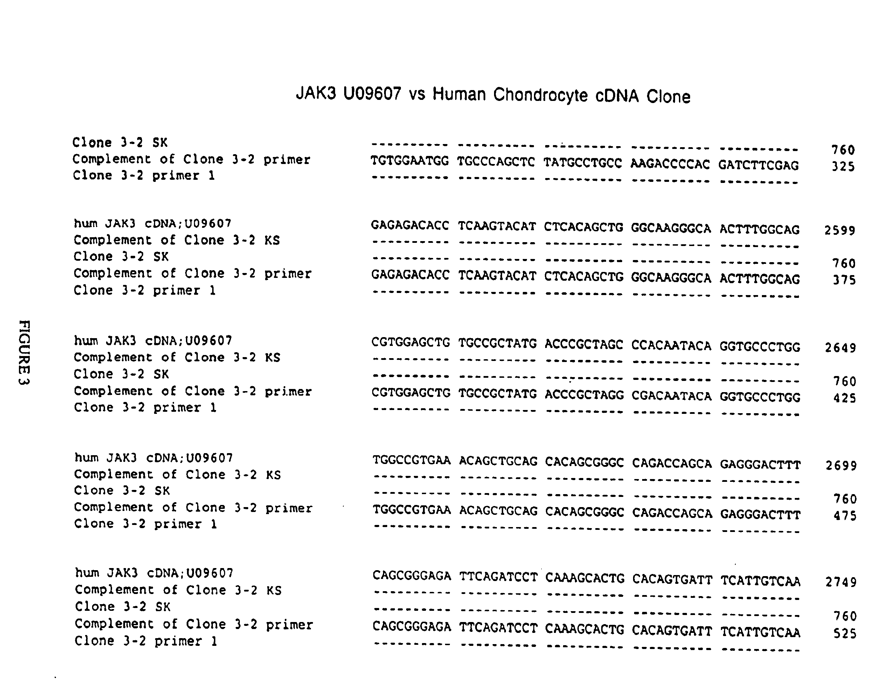

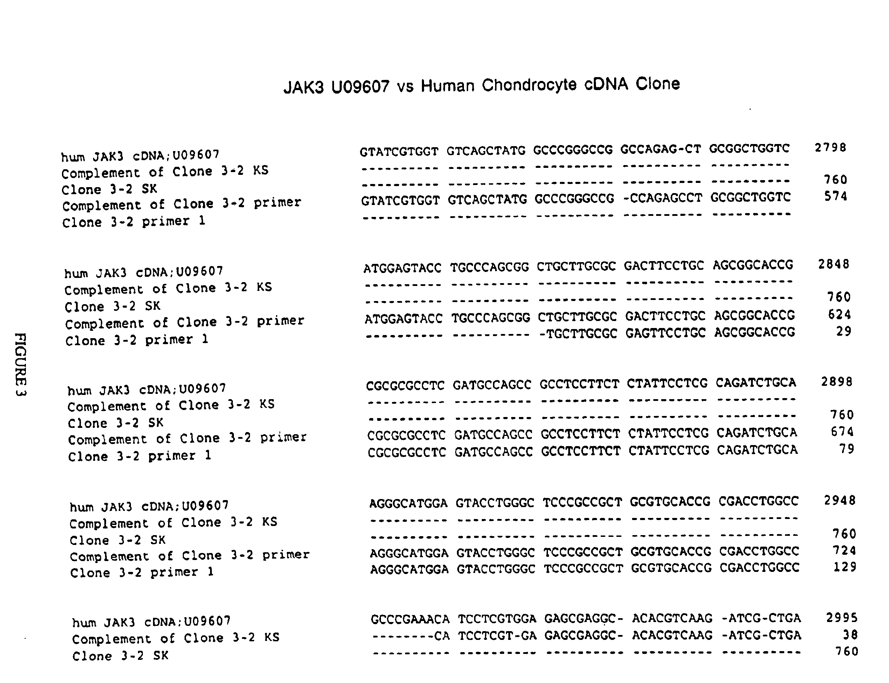

Of the 214 nucleotides sequenced, 197 nucleotides showed 100% identity to the GenBank human JAK3 cDNA sequence (accession number U09607) (FIG. 2). The remaining 17 nucleotide discrepancies were attributed to the suboptimal quality of the sequencing template.

### EXAMPLE 4: Isolation of JAK3 cDNA from a Human Chondrocyte cDNA Library

To further demonstrate that JAK3 is actively expressed in human chondrocytes, JAK3 was isolated from a human chondrocyte cDNA library.

Using techniques known in the art, total RNA isolated from human articular chondrocytes cultured in alginate was used to generate a cDNA library (cDNA library construction was commercially performed by Stratagene, La Jolla, CA). The cDNA library was titered and screened following well established protocols (Sambrook et al., 1989, and the recommended protocols of Stratagene, La Jolla, CA).

A JAK3 DNA probe was used to screen the chondrocyte cDNA library. Prehybridization and hybridization of the cDNA library filters was performed using a 1X Prehybridization/Hybridization Solution (GIBCO/BRL, Gaithersburg, MD) at 65°C. After 15 hours of hybridization with the JAK3 DNA probe, the blot was successively washed two times in 2X SSC/0.1% SDS (15 minutes/wash at room temperature), and two times in 1X SSC/0.1% SDS (30 minutes/wash at 65°C). The blot was allowed to air dry, and the radioactive signals were visualized using Kodak X-Omat™ AR (XAR) autoradiographic film (Eastman Kodak, Rochester, NY).

Plaques that were positive on duplicate filters were picked, placed in 1 ml of sterile SM buffer (0.05 M Tris pH 7.5, 0.1 M NaCl, 0.008 M MgSO₄, 0.01% gelatin), vortexed, and incubated at 4°C overnight. Fourteen positive plaques from the first screening were replated using 10 *µ*l of a 1 x 10⁻² dilution into 200 *µ*l of the bacterial strain XL-1 Blue MRF' (Stratagene, La Jolla, CA), and screened with a JAK3 DNA probe as above. Positive plaques from the second screening were picked and placed in SM Buffer as above.

Two positive phage clones from the second screening were selected for DNA sequence analysis based on; i) confirmation of JAK3 identity using primers JK3-1 and JK3-3 in a PCR reaction as described in Example 2, and ii) identification of the size of the cloned cDNA insert using PCR primers T7 and T3 (Stratagene, La Jolla, CA). The hot start PCR protocol consisted of an initial denaturation for 5 min at 93°C (followed by the addition of Taq polymerase), followed by: denaturation for 1 min at 93°C; annealization for 1 min at 55°C; extension for 45 sec at 75°C. Denaturation, annealization, and extension proceed for 40 cycles, followed by a final extension for 7 min at 75°C. The size of PCR products were visualized in a 1% SeaKem^{®} GTG^{®} agarose gel (FMC BioProducts, Rockland, ME).

After PCR-product identification of the two lambda ZAP® II phage clones, which contained the largest cDNA inserts for JAK3, the JAK3 cDNA inserts were each subcloned into a SOLR *E. coli* strain using the ExAssist™ Interference-Resistant Helper Phage (Stratagene, La Jolla, CA) according to the manufacturer's suggested protocol. Several bacterial colonies were isolated, and plasmid DNA was purified from these clones using a QIAprep Spin Miniprep Kit (Qiagen, Valencia, CA).

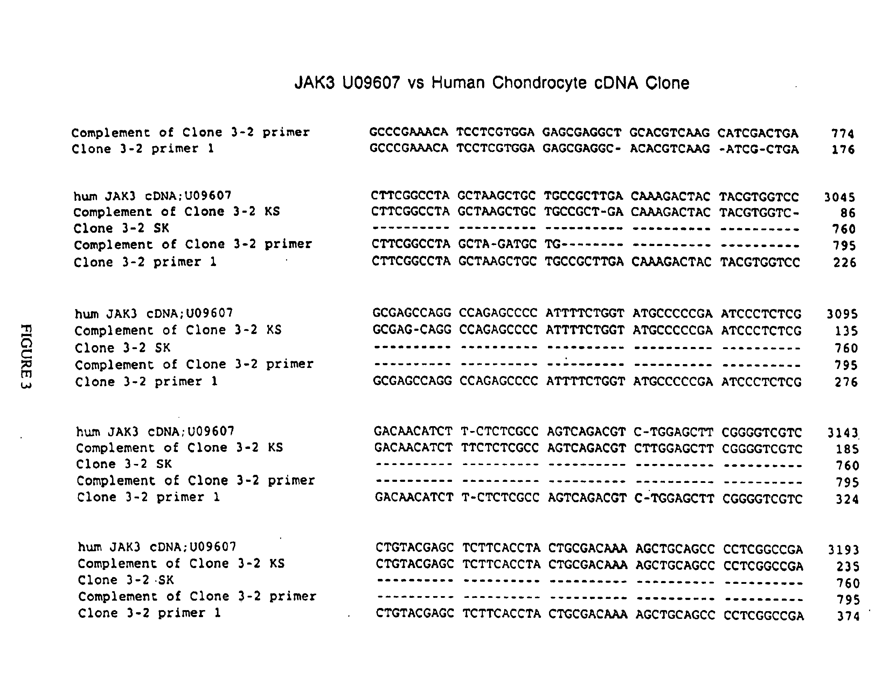

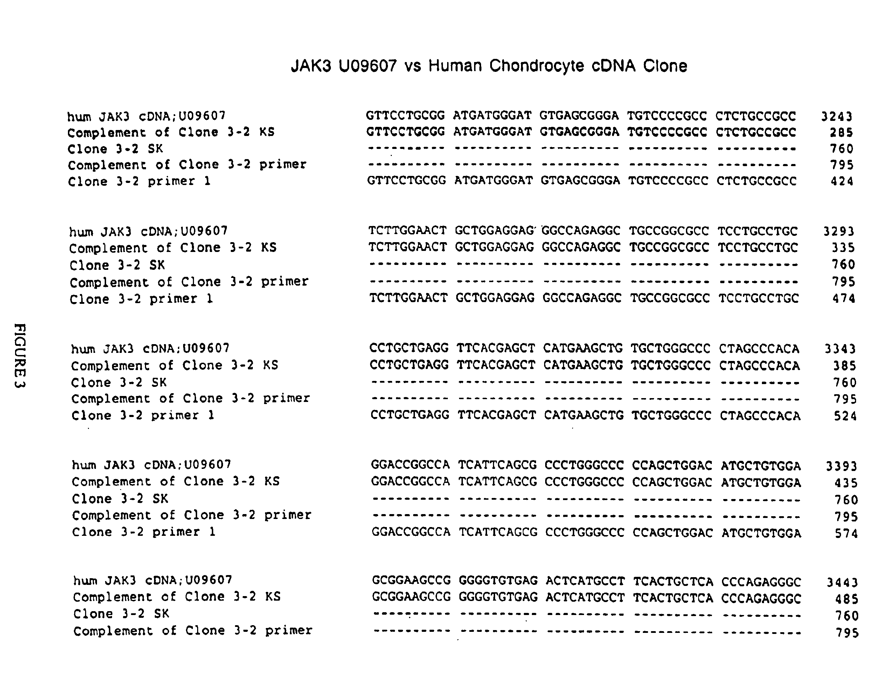

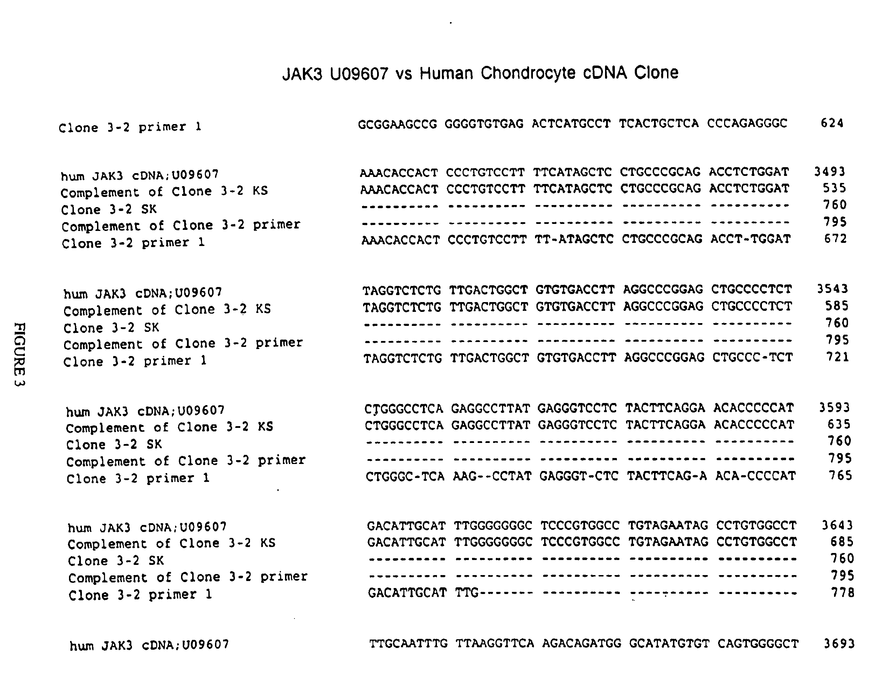

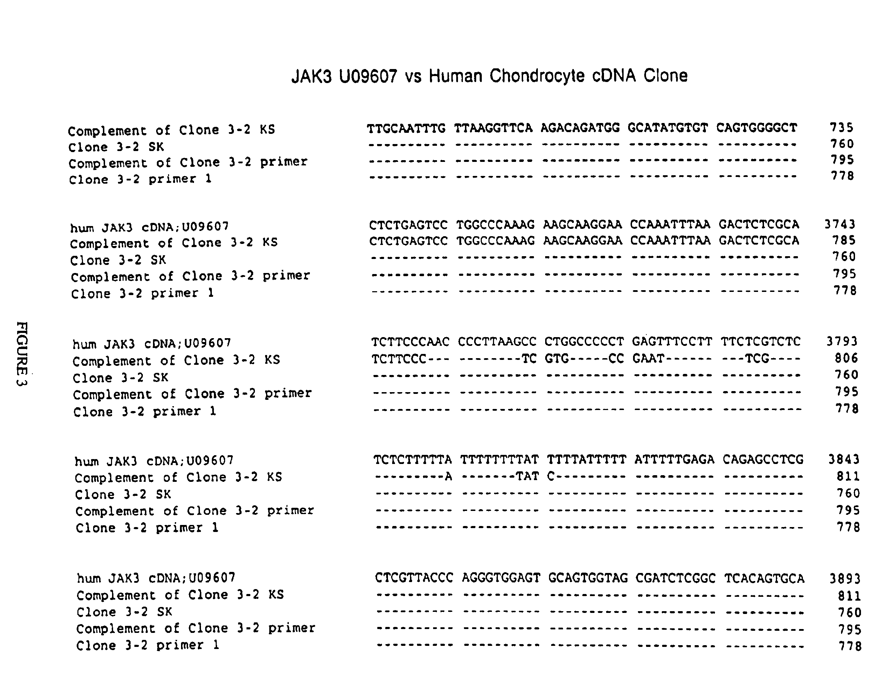

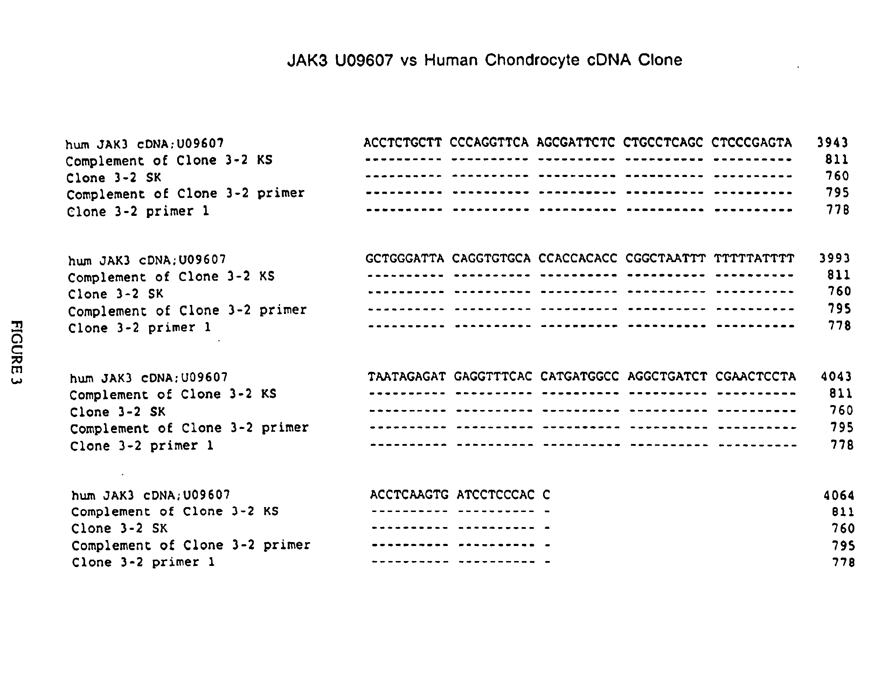

DNA sequence analysis of one DNA strand from each of the two sublcones demonstrated that the approximately 2000 nucleotide sequence that was obtained from each subclone was identical to the published JAK3 cDNA sequence (GenBank Accession #U09607). The DNA sequence determined from one of the subclones is provided as FIG. 3.

Results from Examples 2, 3, and 4 confirm, for the first time, the expression of JAK3 in cultured articular chondrocytes from normal human cartilage, as well as in human osteoarthritic cartilage.

### EXAMPLE 5: Northern Blot Analysis of JAK3 Expression in Human Articular Chondrocytes

Frozen chondrocyte cell strain HC30-0198 (normal human articular chondrocytes prepared as described in Example 1) was thawed, cultured in monolayer (second passage), and cultured to 5 days post confluence. Test cultures were rinsed in phosphate buffered saline (PBS), followed by addition of either; 1) recombinant human interleukin-1β (rhIL-1β) (R&D Systems, Minneapolis, MN) at 2 ng/ml for 24 hours in serum-free DMEM containing 1% antibiotic solution, or 2) serum-free DMEM containing 1% antibiotic solution only (control).

Total RNA from monolayer culture was harvested after 24 hours of culture using TRIzol Reagent (GIBCO/BRL, Gaithersburg, MD) according to the manufacturer's suggested protocol. 10 *µ*g of total RNA from each sample above was separated in a 2.2M formaldehyde /1.2 % agarose gel, and transferred to a nylon support membrane (Schleicher & Schuell, Keene, NH) by mild alkaline transfer using the TURBOBLOTTER^{™} (Schleicher & Schuell, Keene, NH) system according to the manufacturer's suggested protocol. RNA on the Northern blot was fixed to the membrane by using a Stratatinker^{®} 1800 UV Crosslinker (Stratagene, La Jolla , CA) .

A 109-base pair human cDNA for JAK3 was generated by RT-PCR (see Example 2 above). The cDNA was labeled with [α-³²P] dCTP (New England Nuclear, Boston, MA) using the Ready-To-Go DNA Labeling Beads (-dCTP; Amersham Pharmacia Biotech, Piscataway, NJ) according to the manufacturer's suggested protocol, purified using CHROMA SPIN^{™} +TE-30 Columns (Clontech, Palo Alto, CA), and used as a probe for a Northern-blot analysis.

Prehybridization and hybridization of the Northern blot was performed at 42°C using a 1:1 dilution of 2X Prehybridization/Hybridization Solution (GIBCO/BRL, Gaithersburg, MD) and formamide. After 15 hours of hybridization using the JAK3 DNA probe, the blot was successively washed two times in 2X SSC/0.1% SDS (15 minutes/wash at room temperature), and two times in 0.5X SSC/0. 1% SDS (30 minutes/wash at 65°C). The blot was allowed to air dry, and the radioactive signal was visualized using a Fujifilm BAS-1500 phosphorimager (Fuji Medical Systems, USA, Stamford, CT).

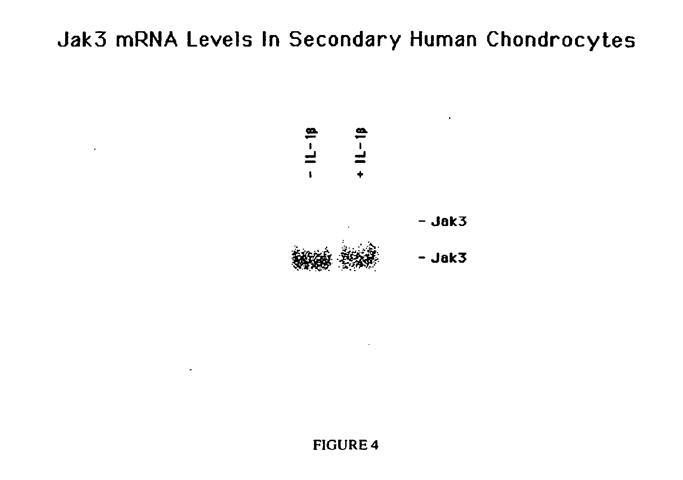

The phosphoimage of northern blot analysis is provided as FIG. 4. The results demonstrate that JAK3 mRNA is expressed in cultured normal adult human articular chondrocytes, and that rhIL-1β neither increases nor decreases the level of JAK3 mRNA found in non-rhIL-1β-stimulated normal adult human articular chondrocytes. In addition, the northern blot reveals that two molecular weight species of JAK3 are expressed by human articular chondrocytes in monolayer culture: approximately 4.2 kb and 2.2 kb in length. The predominant form of JAK3 mRNA expressed by cultured normal adult human articular chondrocytes is the 2.2 kb form. The larger (4.2 kb) form is consistent in size with that found in normal human blood cells of lymphoid lineage, and represents a small fraction of the total JAK3 mRNA expressed in chondrocytes.

### EXAMPLE 6: DBH Inhibition of JAK3 (reference)

To demonstrate that DBH, an effective therapeutic for degenerative joint disease such as OA, functions to inhibit JAK3, a tyrosine kinase enzyme-linked immunosorbent assay (ELISA) was performed.

Nunc-Immunoplate Maxisorp flat-bottomed capture plates (Nalge Nunc International, Rochester, NY) were coated with 80 µl/well anti-phosphotyrosine PY54 MAb antibody (Transduction Labs, Lexington, KY), diluted to 2 µg/ml in coating buffer (10 mM phosphate, pH 7.2 + 0.02% NaN₃), and allowed to adsorb overnight at 4°C. The plates were washed (all washes are 4X with TBST: 25 mM Tris/HCl, pH 8.0, 150 mM NaCl + 0.05% Tween-20), and 200 µl/well of blocking buffer (TBST + 1% BSA) was added. The plates were sealed and incubated at 37°C for 60 min or overnight at 4°C, and washed again.

Kinase reactions were run in 50 µl volumes with biotinylated peptide substrate in a roundbottom plate (Coming, Corning, NY). Kinase reactions mixtures contained; 20 µl 50 µM GAS1 biotinylated peptide (LCBiotin-EGPWLEEEEEAYGWMDF-amide), 0 - 1250 µM ATP in kinase buffer, 10 µl 50 mM MgCl₂ in 2X kinase buffer (50 mM imidazole/HCl, 2 mM DTT, 0.2 mM EDTA, 0.030% Brij-35, pH 6.8), 10 µl 0 - 50 µM DBH in water containing 0.2% Pluronic-104 and 3% dimethyl sulfoxide (DMSO), and 10 µl JAK3 enzyme diluted in kinase buffer. A phosphopeptide standard curve (0 - 10 nM) using kinase buffer as the diluent was also tested.

Kinase reactions were run for 30 min at 37°C, then stopped by adding 15 *µ*l/well of 0.125 M EDTA, pH 6.8. Aliquots of 50 *µ*l/well of stopped solutions were transferred to an antibody-coated capture plate, sealed, and incubated at 37°C for 60 min. The plate was washed, and 80 *µ*l/well of SA-HRP reagent (Genzyme, Cambridge, MA) diluted 1/5 in Genzyme dilution buffer was added. The plate was sealed and incubated again at 37°C for 60 min. The plate was washed, and 100 *µ*l/well of tetramethyl benzidine (TMB) substrate solution (Kirkegaard & Perry Laboratories, Gaithersburg, MD), pre-equilibrated to room temp was added, and allowed to react for approximately 5 min., The reaction was stopped with 100 µl/well of IN H₂SO₄ solution. Samples were read at A₄₅₀-A₆₂₀.

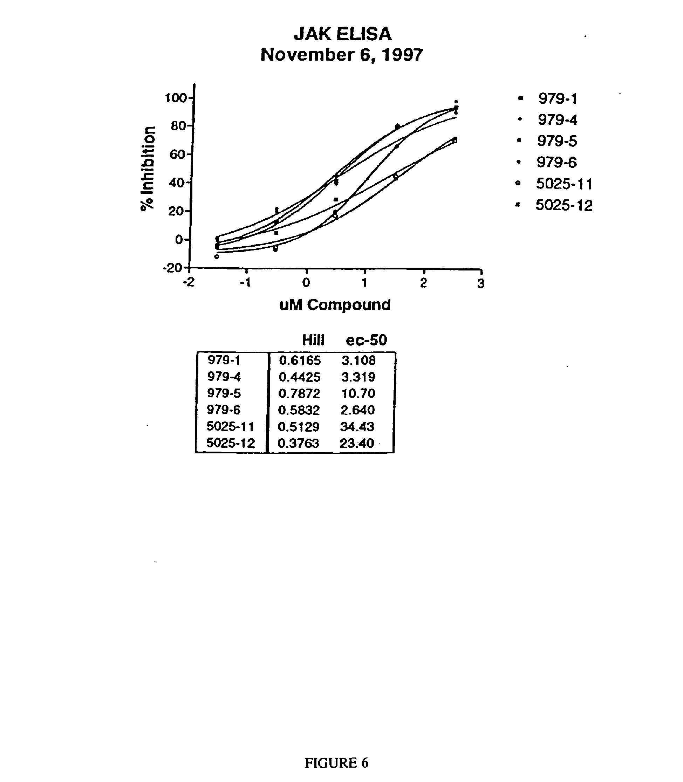

The results of the experiment (provided as FIG. 6) reveal DBH is a strong inhibitor of JAK3, with a recorded inhibition constant (Kᵢ) of 286 ± 16 nM.

### EXAMPLE 7: DBH (and variants thereof) Specifically Inhibits JAK3 (reference)

To further examine the inhibition characteristics of DBH (and its variants) to a variety of cellular components, a poly (Glu, Tyr) 4:1 ELISA was performed. Hymenialdisine (H), DBH (as marine sponge extracts: see Chipman and Faulkner, 1997), and three variant forms of synthetic DBH: the free base form of DBH (DBH-2FB), a trifluoroacetic acid salt form of DBH (DBH-2S), and a methanesulfonic acid salt form of DBH (DBH1) were solubilized in DMSO, and tested for the ability to inhibit; ZAP-70 (ζ-associated polypeptide of 70 kDa) protein tyrosine kinase, BTK (Bruton's agammaglobulinaemia tyrosine kinase), JAK3 (Janus kinase 3) protein tyrosine kinase, LCK (Lymphoid T-cell protein tyrosine kinase), and IGFR (Insulin-like growth factor receptor) protein tyrosine kinase.

Maxisorb microtiter plates (Nalge Nunc International, Rochester, NY) were coated with 1 mg/ml Poly(Glu,Tyr) 4:1 (Sigma, St. Louis, MO) solubilized in phosphate buffered saline (PBS), 0.02% sodium azide. On the day of the assay, microtiter plates were washed 4X with TBS-Tween (25 mM Tris pH 8.0,150 mM NaCl, 0.05% Tween 20) using a Skatron plate washer (Skatron Instruments, Sterling, VA). 200 µl of sterile blocking buffer (3% BSA in PBS, 0.02% sodium azide) was added to each well, and the microtiter plates were incubated at 37°C for 1 hour. The blocking buffer was removed, the plates washed, and various concentrations of inhibitor were added in a 20 *µ*l volume to the wells. 60 *µ*l of the appropriate substrate mix was then added. Substrate mixes were prepared as described in Table 2:

**Table 2: Poly (Glu, Try) 4:1 ELISA Substrate solution.**

| | 4X Assay Buffer* | ATP | MnCl₂ | MgCl₂ | H₂O |
|---|---|---|---|---|---|
| ZAP-70 | 2 ml | 1 ml 500 µM | 1 ml 100 mm | -- | 2 ml |
| BTK | 2 ml | 1 ml 500 µM | 1 ml 100 mm | 1 ml 10 mM | 1 ml |
| JAK3 | 2 ml | 1 ml 500 µM | -- | 1 ml 100 mM | 2 ml |
| LCK | 2 ml | 1 ml 500 µM | 1 ml 100 mM | 1 ml 100 mM | 1 ml |
| IGFR | 2 ml | 1 ml 500 µM | 1 ml 100 mm | 1 ml 50 mM | 1 ml |

| | | | | | |
|---|---|---|---|---|---|
| *(4X assay Buffer is 100 mM imidazole pH 6.8, 0.4 mM EDTA, 4 mM DTT, 0.06% Brij-35) | | | | | |

Following addition of the substrate mix, 20 *µ*l of the test protein tyrosine kinase (in 1X assay buffer) was added. Kinase reactions were incubated 30 min at 37°C, followed by washes in the Skatron plate washer. 100 µl of biotinylated PT-66 anti-phosphotyrosine monoclonal antibody (Sigma, St. Louis, MO) (diluted 1:4000 in 0.5% BSA, sterile-filtered TBS) was added to each well, and incubated for an additional 30 minutes at 37°C. Plates were washed, and 100 µl of streptavidin-HRP conjugate (ICN Pharmaceuticals, Costa Mesa, CA) diluted 1:32,000 in 0.5% BSA-sterile-filtered TBS was added to each well, and incubated for a final 30 minutes at 37°C. Plates were washed, and 100 *µ*l of tetramethyl benzidine (TMB) substrate solution (Kirkegaard & Perry Laboratories, Gaithersburg, MD) was added for 15 minutes at room temperature, followed by addition of 100 *µ*l of Stop Reagent (1 M phosphoric acid). The samples were then read at 450 nm in a plate reader.

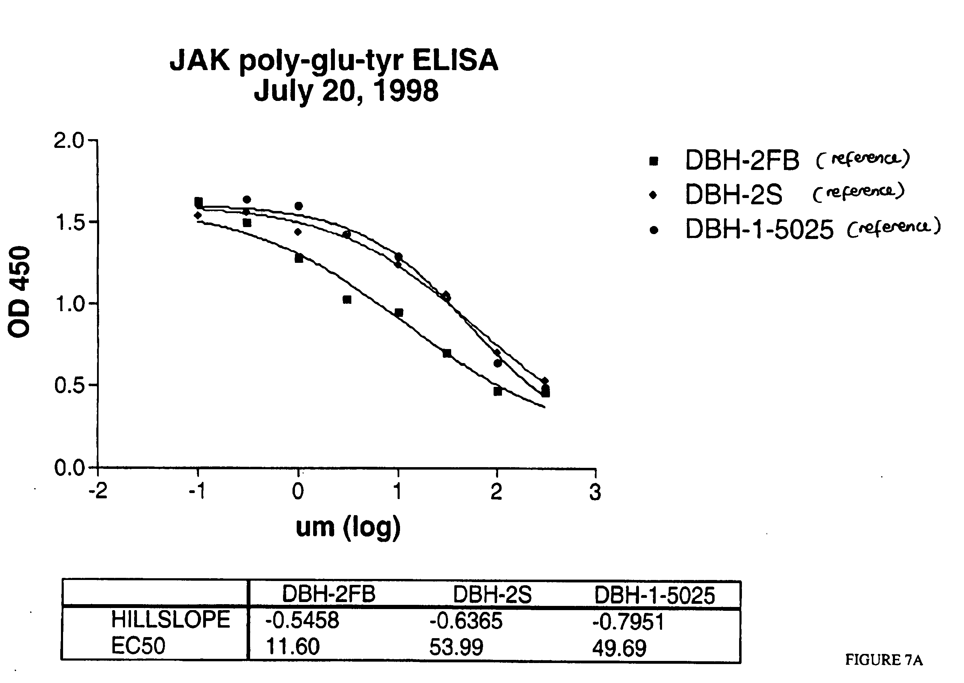

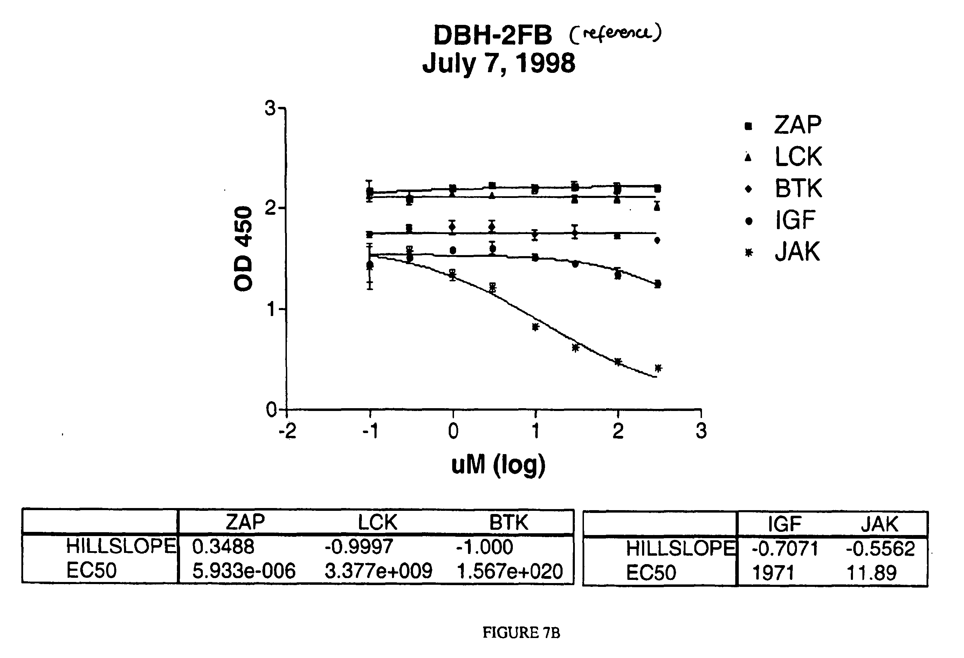

Four lots of the marine extract H (referred to as 979-1, 979-4, 979-5, 979-6) and two lots of the marine extract DBH (referred to as 5025-11 and 5025-12) showed specific low micromolar inhibition of JAK3, with ec-50 values (effective concentration for 50% inhibition) ranging from 2.6 *µ*M - 10.7 *µ*M (979 lots) and 23.4 *µ*M- 34.4 *µ*M (5025 lots) (FIG. 7). Inhibition of ZAP-70, LCK, BTK, and IGFR was weak, with ec-50 values ranging from hundreds of micromolar, to no inhibition (data not shown).

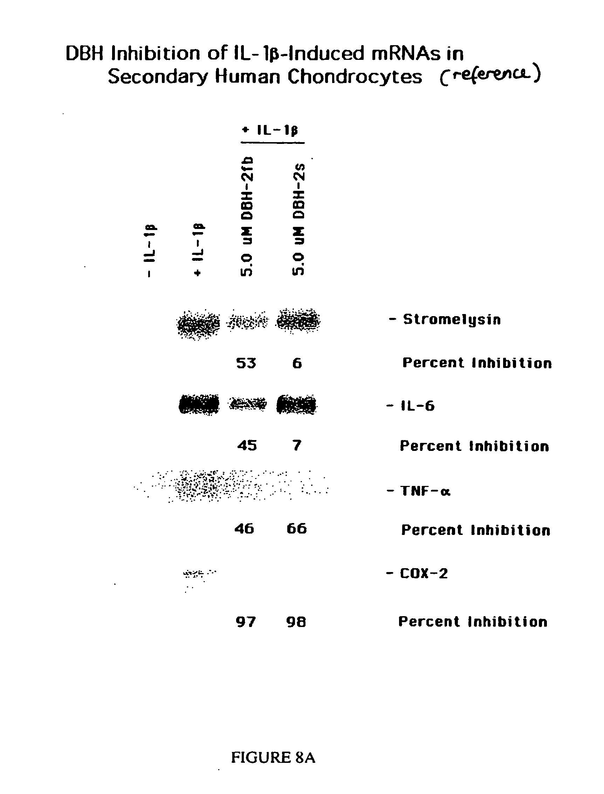

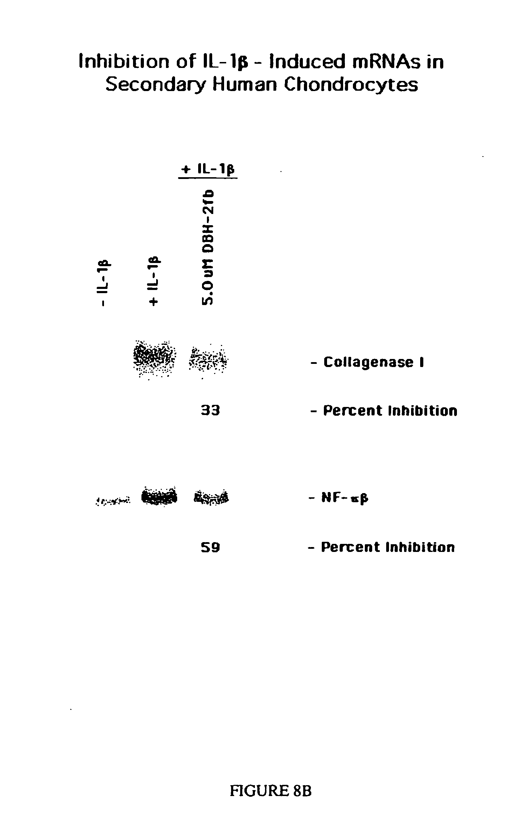

The three forms of synthetic DBH tested also showed inhibition of JAK3, with ec-50 values ranging from 11.60 *µ*M - 53.99 *µ*M (FIG. 8A). DBH-2FB demonstrated the greatest inhibition of JAK3 when compared with the two salt forms of DBH (i.e. 11.60 *µ*M). DBH-2FB is also shown to be a selective inhibitor of JAK3, with ec-50 values for ZAP-70, LCK, BTK, and IGFR ranging from 1971 *µ*M to no inhibition (FIG. 8B).
These results demonstrate DBH and its various derivatives to be JAK3-specific inhibitors.

### EXAMPLE 8: Down Regulation of OA-Associated mRNAs by DBH (and variants thereof) (reference)

To examine the downstream effects of JAK3 inhibition by DBH, northern blot assays were performed to detect changes in steady state mRNA levels of various OA-associated molecules.

Frozen human articular chondrocyte cell strain HC30-0198 (described in Example 1) was thawed, and cultured in monolayer in T150 tissue culture flasks (Coming Costar, Cambridge, MA) as described above to 1-5 days post-confluence. Test cultures were rinsed in PBS, and preincubated for 2 hours with 10 ml of serum-free DMEM containing 5 µM DBH (synthetic), in its free base or trifluoroacetic acid salt form. An additional 10 ml of serum-free DMEM containing 5 µM DBH, 4 ng/ml recombinant human interieukin-1β (rhIL-1β; final concentration of 2 ng/ml) (R&D Systems, Minneapolis, MN), and 2% antibiotic solution (final concentration of 1%), were then added for 24 hours.

Two control cultures were run in parallel: one without DBH (rhIL-1β alone), and another without DBH or rhIL-1β.

Total RNA from monolayer culture was harvested using TRIzol Reagent (GIBCO/BRL, Gaithersburg, MD) according to the manufacturer suggested protocol. 10 *µ*g of total RNA from each test sample above was separated in a 2.2M formaldehyde / 1.2 % agarose gel, and transferred to a nylon support membrane (Schleicher & Schuell, Keene, NH) by mild alkaline transfer using the TURBOBLOTTER^{™} system (Schleicher & Schuell, Keene, NH) according to the manufacturer's suggested protocol. Total RNA was fixed to the membrane using a Stratalinker® 1800 UV Crosslinker (Stratagene, La Jolla, CA).

Human DNA probes for stromelysin-1 (MMP3), collagenase I (MMP1), cyclooxygenase II (COX2), NF-κB (p65), tumor necrosis factor-α (TNF-α), and interleukin-6 (IL-6) were labeled with [α-³²P] dCTP (New England Nuclear, Boston, MA) using the Ready-To-Go DNA Labeling Beads (-dCTP) (Amersham Pharmacia Biotech, Piscataway, NJ) according to the manufacturer's suggested protocol. The radioactive probes were separated from unincorporated [α-³²P] dCTP using CHROMA SPIN^{™} +TE-30 Columns (Clontech, Palo Alto, CA), and purified probes were used for northern blot hybridization experiments.

Prehybridization and hybridization of the Northern blots with the radioactive probes were performed using ExpressHyb^{™} Hybridization Solution (Clontech, Palo Alto, CA) according to the manufacturer's suggested protocol. The blot was successively washed two times in 2X SSG0.05% SDS (15 min/wash at room temperature), and two times in 0.1X SSC/0.1% SDS (30 minutes/wash at 65°C). The blot was allowed to air dry, and the radioactive signal was visualized and quantified using a Fujifilm, BAS-1500 phosphorimager (Fuji, Stamford, CT).

Normalization of RNA loading was performed by ethidium bromide staining of the northern blot. 5 *µ*l of 10 mg/ml ethidium bromide (Sigma, St. Louis, MO) was added to 50 ml of 1X MOPS solution (MESA buffer: Sigma, St. Louis, MO), and stained for 7 min at room temperature. The blot was washed for 30 min (3 times) with 1X MOPS buffer. The ethidium bromide staining pattern was visualized on a Fujifilm LAS-1000 Intelligent Dark Box (Fuji Medical Systems, USA, Stamford, CT).

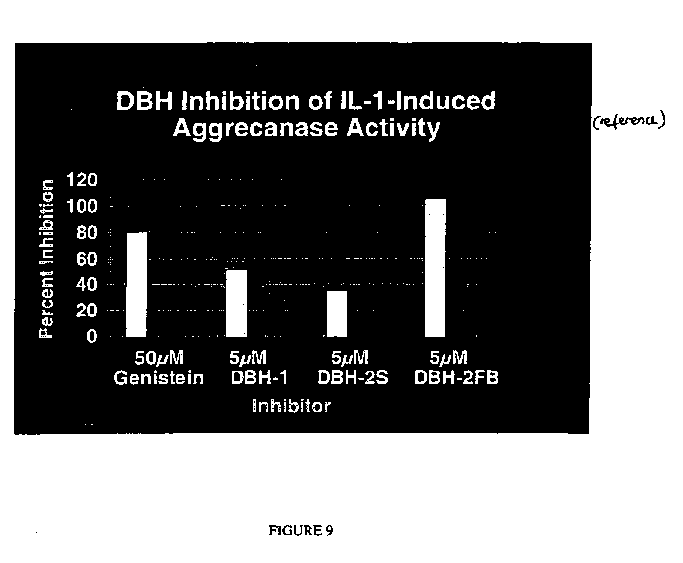

The results of the northern blot experiments (shown in FIGs 9A and 9B) demonstrate that 5 µM of the JAK3-inhibitor DBH was able to inhibit to various degrees the IL-1β-induced upregulation of mRNAs known, or believed, to be associated with the pathology of (osteo)arthritis: stromelysin (MMP3), collagenase 1 (MMP1), cyclooxygenase II (COX2), NF-κB (p65), tumor necrosis factor-α(TNF-α), and interleukin-6 (IL-6).

Of interest is the unexpected observation that the free base form of DBH inhibit rhIL-1β-induced mRNAs to a greater extent than the trifluoroacetic acid salt form of DBH for some mRNAs (FIG. 9A). This observation is consistent with the data obtained in the tyrosine kinase assays of Example 7.

### EXAMPLE 9: DBH Inhibition of IL-1α-Induced Aggrecanase Activity in Bovine Chondrocytes (reference)

To demonstrate the cellular effects of DBH as a JAK3-specific inhibitor, inhibition of DBH and its variants was measured by IL-1α-induced ³⁵S-labeled proteoglycan release in bovine chondrocytes (It is well established that IL-1α-induced proteoglycan release in cultured primary bovine chondrocytes and cartilage explants is due to cleavage of aggrecan by the enzyme known as aggrecanase).

Primary bovine articular chondrocytes were isolated from the dissected articular cartilage of calf stifle joints using a thirty minute (37°C) 1 mg/ml hyaluronidase (Boehringer Mannheim, Indianapolis, IN) treatment, followed by a thirty minute (37°C) 2 mg/ml collagenase P (Boehringer Mannheim, Indianapolis, IN) and 2.5 mg/ml trypsin (GIBCO/BRL, Gaithersburg, MD) treatment. Enzyme solutions were prepared in serum-free 1:1 Delbecco's modified essential medium/Harris F-12 (DMEM/F12), and filter sterilized with a 0.22 µm Millex^{®}-GV filter (Millipore S.A., Molsheim, France). A third, overnight digestion ( 0.5 mg/ml collagenase P at 37°C) was performed in a Bellco stir flask.

Bovine chondrocytes were recovered by addition of an equal volume of DMEM/F12 supplemented with 10% fetal bovine serum (FBS) to neutralize enzymes, filtered through a 70 µm nylon Falcon^{®} Cell Strainer (Beckton Dickinson, Franklin Lakes, NJ), and centrifuged at 1000X g for 10 min at room temperature.

Chondrocytes were seeded in Costar^{®} 24-well tissue culture plates (Coming, Corning, NY) at 8 x 10⁴ cells/well using 0.5 ml of 1:1 DMEM/F12, 10% FBS, 1% antibiotic solution (penicillin, streptomycin, fungizone) (GIBCO/BRL, Gaithersburg, MD), and incubated at 37°C, 8% CO₂. Cells were cultured for 28 days with refeeding on every third day with DMEM/F12 plus 10% FBS, 1% antibiotic solution.

Metabolic labeling of proteoglycans was performed on day 22 with 10 µCi of ³⁵Sulfate (Amersham, Arlington Heights, IL) in serum-free DMEM/F12, 1% antibiotic solution for 48 hours. The free ³⁵Sulfate was removed on day 24, chondrocytes were washed with PBS, and were refed with DMEM/F12 plus 10% serum, 1% antibiotic solution. Chondrocytes were cultured for an additional two days, refed on day 26 with DMEM/F12 plus 10% serum, 1% antibiotic solution, and were washed on day 27 with PBS.

After the PBS wash, chondrocytes were treated with 0.5 ml of serum-free DMEM/F12, 1% antibiotic solution containing either; 1) no further supplements, or 2) 5 µM DBH (as either DBH1, DBH-2S, or DBH-FB). After 2 hours, rhIL-1α was added to the cultures (except for the "no rhIL-1α"control) to a final concentration of 1 ng/ml.

On day 28, the 0.5 ml media was removed and placed in a scintillation minivial. 4 ml of scintillation fluid was added and radioactivity in the media was quantified by scintillation counting. The cell layer was rinsed in PBS and harvested with 0.5 ml of 1X trypsin-EDTA, and quantified by scintillation counting as above. All samples were cultured in triplicate.

Data were determined and expressed as follows:
- 1. Background Fraction_{c}: = Fraction of total radioactivity (media + cell layer) that represents the amount of non-IL-1-induced radioactivity released into the media.
= [total CPMs in media of the "No IL-1α, No DBH Control"/(total CPMs in media of the No IL-1α, No DBH Control"+ total CPMs in cell layer of the "No IL-1α, No DBH Control")]
- 2. % Release of ³⁵S-labeled proteoglycan into media by IL-1α: a) Background = Counts in the media of test sample due to non-IL-1-induced release
= Background Fraction_{C} [total CPM in test sample media + total CPM in test sample cell layer]
b) CPM released by IL-1α = Total CPM in media - Background
c) % Release by IL-1α = [CPM released by IL-1α/(CPM released by IL-1α + total CPM in cell layer)] 100 %
- 3. % Release by IL-1α in the presence of DBH: = {(Total CPM in DBH media sample - Background) / [(Total CPM in DBH media sample - Background) + total CPM in DBH cell layer sample]} 100%
- 4. % Inhibition by DBH: = [I - (% Release by IL-1α in the presence of DBH / % Release by IL-1α] 100%

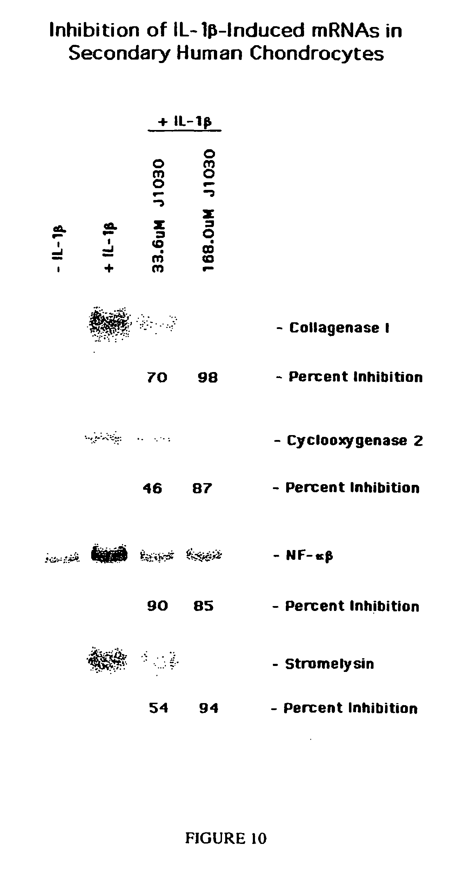

The three forms of the synthetic DBH (i.e. DBH1, DBH-2S, or DBH-FB) showed various degrees of inhibition of IL-1α-induced ³⁵S-labeled proteoglycan release: - 50%, ~ 40%, and 100%, respectively (FIG. 10). Once again the free base form of DBH as an inhibitor produced results dramatically superior to that of the other two forms of DBH.

### EXAMPLE 10: Down Regulation of OA-Associated mRNAs by Compound WHI-P131

To demonstrate that DBH and its related compounds operate as an effective OA therapeutic by functioning as a JAK3-specific inhibitor, and to confirm the effectiveness JAK3 inhibitors generally as inhibitors of OA-associated compounds and thus as a novel class of OA therapeutics, inhibition of various OA-associated mRNAs by the well known JAK3-specific inhibitor 4-(4'-hydroxyphenyl)-amino-6,7-dimethoxyquinazoline (also referred to in the art as WHI-P131) was evaluated.

Normal human articular chondrocytes were isolated from cartilage slices and cultured as described in Example 1. mRNA inhibition experiments were performed identical to the protocols described in Example 8, with the exception that the JAK3-specific inhibitor, WHI-P131(4-(4'-hydroxyphenyl)-amino-6,7-dimethoxyquinazoline, purchased from A.G. Scientific, San Diego, CA), was used in place of DBH. Two concentrations of WHI-P131, solubilized in DMSO, were tested: 33.6 *µ*M and 168 *µ*M.

The results of the northern blot experiment (shown in FIG. 11) demonstrate that both the 33.6 *µ*M and 168 *µ*M treatment of the JAK3-specific inhibitor, WHI-P131, inhibit the IL-1β-induced upregulation of mRNAs known, or believed, to be associated with the pathology of (osteo)arthritis: i.e., stromelysin-1 (MMP3), collagenase 1 (MMP1), cyclooxygenase II (COX2), and NF-kB (p65).

The present invention refers to techniques well known in the field of molecular biology, which are described in the following publications:
Old, R.W. & S.B. Primrose, Principles of Gene Manipulation: An Introduction To Genetic Engineering (3d Ed. 1985) Blackwell Scientific Publications, Boston. Studies in Microbiology; V.2:409 pp. (ISBN 0-632-01318-4).
Sambrook, J. et al. eds., Molecular Cloning: A Laboratory Manual (2d Ed. 1989) Cold Spring Harbor Laboratory Press, NY. Vols. 1-3. (ISBN 0-87969-309-6).
Winnacker, E.L. From Genes To Clones: Introduction To Gene Technology (1987) VCH Publishers, NY (translated by Horst Ibelgaufts). 634 pp. (ISBN 0-89573-614-4).

### References

Abbaszade et al., 1999. J Biol. Chem. 274(33):23443-23450.
Alaaeddine et al., 1997. Arthrtis Rheum. 40:839.
Aman and Leonard, 1997. Current Biology 7:R784-788.
Argetsinger et al., 1993. Cell 74:237.
Aringer ct al., 1999. Life Sci. 64(24):2173-2186.
Arner et al., 1997. J. Biol. Chem. 272(14):9294-9299.
Asahara et al., 1997. Arthritis Rheum. 40: 912-918.
Attur et al. 1998. Proc. Assoc. Am. Physicians 110(1):65-72.
Blank et al., 1989. Nature 337:187-189.
Buckley et al., 1997. J. Pediatr. 130:378-387.
Buttle et al., 1993. Arth. Rheum. 12:1709.
Caron et al., 1996. Arthris Rheum. 39:1535-1544.
Chen et al., 1997. PNAS USA 94:6910-6915.
Chen et al., 1999. Pharm. Res. 16(1): 117-122.
Chipman and Faulkner, US PAT NO. 5,591,740 (7 January 1997).
Chung et al., 1997. Science 278:1803-1805.
Civin et al. US PAT NO. 5,705,625 (6 January 1998).
Civin et al. US PAT NO. 5,916,792 (29 June 1999).
Costello et al. 1996. Oncogene 13:2595-2605.
Danial et al., 1995. Science, 269:1875-1877.
Darnell et al., 1994. Science 264:1415-1421.
Darnell, 1997. Science 277:1630-1635.
Dean et al., 1989 J. Clin. Invest. 84:678-685.
De-Fraja et al., 1998. J. Neurosci. Res. 54(3):320-330.
Disanto et al., 1995. PNAS USA 92:377-381.
Endo et al., 1997. Nature 387: 921-924.
Farrell et al. 1992. Ann. Rheum. Dis. 51:1219-1222.
Fernandes et al., 1997. Arthritis Rheum. 40:284-294.
Firestein et al., 1991. Arthritis Rheum 34: 1094-1105.
Firestein, 1996. In: Textbook of Rheumatology. Kelly et al. (eds.) 5th edition pp. 851-897.
Firestein and Manning, 1999. Arthritis Rheum. 4: 609-621.
Firmbach-Kraft et al., 1990. Oncogene 5:1329.
Galli, 1993. N. Engl. J. Med. 328:257-265.
Gauzzi et al., 1996. J. Biol. Chem. 271:20494-20500.
Gazit et al., 1989. J. Med. Chem. 32:2344-2352.
Goldring, 1999. Connective Tissue Res. 401(1):1-11.
Goodman et al., 1998. J. Biol. Chem. 273:17742-17748.
Gordon and Galli, 1990. Nature 346:274-276.
Gurniak and Berg, 1996. Blood 87(8):3151-3160.
Hamaway et al., 1995. Cell. Signalling 7:535-544.
Han et al., 1998. Autoimmunity 28:197-208.
Hanks et al., 1991. Meth. Enzymol. 200:38.
Harpur et al., 1992. Oncogene 7:1347.
Helden and Purton (eds.) 1996. In: Signal Transduction pp. 19-31.
Henderson and Pettipher, 1989. Clin. Exp. Immunol. 75:306-310.
Hilton et al., 1998. PNAS USA 95:114-119.
Home, 1997. J. Org. Chem. 62: 456.
Hou et al., 1996. Cell 84:411-419.
Huang et al., 1999. Biochem J. 342(ptl):231-238.
Hunter, 1991. Meth. Enzymol. 200:3.
Ihle et al., 1994. TIBS 19:222-227.
Ihle and Kerr, 1995. Trends Genet. 11: 69-74.
Ihle, 1995. Nature 377:591-594.
Ihle, 1996. Philos. Trans. R. Soc. Lond-Biol. Sci. 351:159-166.
Ihle, 1996. Cell 84(3):331-334.
Izuhara et al., 1996. J. Biol. Chem. 271:619-622.
Johnston et al., 1995. J. Biol. Chem. 270:28527-28530.
Joosten et al., 1997. Arthrtis Rheum. 40:249-260.
Karin et al. 1997. Curr Opin Cell Biol 9:240-246.
Kawamura et al., 1994. PNAS USA 91:6374-6378.
Keegan et al., 1995. PNAS USA 92:7681-7685.
Kirken et al., 1999. J. Leukoc. Biol. 65:891-899.
Kolenko et al., 1999. Blood 93(7):2308-2318.
Kumar et al., 1996. Oncogene 13:2009-2014.
Lai et al., 1995. J. Biol. Chem. 270:25028-25036.
Lee et al., 1994. Nature 372:739-746.
Lefebvre et al., 1990. Biochem. Biophys. Res. Comm. 152:366.
Levitzki and Gazit. 1995. Science 267:1782-1788.
Levy, 1997 Cytokine Growth Factor Rev. 8:81-90.
Liu et al., 1997. Curr. Biol. 7(11):817-826.
Lohmander et al., 1993. Arthritis Rheum. 36:181-189.
Luttichen et al., 1994. Science 263:89.
Luo et al., 1995. EMBO J. 14:1412-1420.
McCachren et al., 1990. J. Clin. Immuno. 10:19-27.
McCartney-Francis et al. 1993. J.Exp. Med. 178:749-754.
Macchi et al., 1995. Nature 377:65-68.
McDonnell, et al., 1992. Arth. Rheum., 35:799.
Magrassi et al., 1999. J. Neurosurg. 91(3):440-446.
Malaviya et al., 1993. J. Biol. Chem. 268:4939-4944.
Malaviya et al., 1999. J. Biol. Chem. 274(38):27028-27038.
Malaviya and Uckun, 1999. Biochem. Biophys. Res. Commun. 257:807-813.
Mankin, 1971. Orthopedic Clinics of North America 2: 19-30.
Mankin and Brandt, 1991. In: Textbook of Rheumatology, Kelly, et al., (eds.) 3rd edition, pp.14699-111471.
Matrisian, 1994. Ann. N.Y. Acad. Sci. 732:42-50.
Matsumoto et al., 1997. Blood 89:3148-3154.
Meydan et al., 1996. Nature 379: 645-648.
Migon et al., 1995. Science 269:79-81.
Minden and Karin, 1997. Biochim Biophys Acta 1333:F85-F104.
Miyazaki and Taniguchi, 1996. Cancer Surveys 27:25-39.
Mort, et al., 1993. Matrix, 13:95.
Mow et al., 1992. Biomaterials 13:67-97.
Muller et al., 1993. Nature 366:129.
Murphy et al., 1987. Biochem. J. 248:265-268.
Musso et al., 1995. J. Exp. Med. 181:1425-1431.
Nambi and Pantil. WO 93/16703 (2 September 1993).
Nambi and Pantil. US PAT NO. 5,616,577 (1 April 1997).
Nelson et al., 1996. Mol. Cell. Biol. 16:369-375.
Nicholson and Hilton, 1998. J. Leukocyte Biol. 63:665-668.
Naka et al., 1997. Nature 387:924-929.
Nosaka et al., 1995. Science 270:800-802.
Ohya et al., 1997. J. Biol. Chem. 272:27178-27182.
Oliver et al., 1994. J. Biol. Chem. 269:29697-29703.
Page-Thomas, 1991. Ann. Rheum. Dis. 50:75-80.
Pelletier, et al., 1991. Sem. Arth. Rheum. 20:12.
Pelletier, et al., 1993. Am. J. Pathol. 142(1):95-105.
Pelletier et al. 1997. In: Arthritis an Allied Conditions: A Textbook of Rheumatology Vol 2. Koopman (ed.) 13th edition, pp. 1969-1984.
Pelletier et al., 1992. In: Cartilage degradation: Basic and Clinical Aspects. Howell and Woessner (eds). pp.503-528.
Pelletier et al., 1993. In: Osteoarthritis, Edition of Rheumatic Disease Clinic of North America. Moskowitz (ed.) pp.545-568.
Pendas et al. 1997. Genomics 40:222-233.
Reboul et al., 1996. J. Clin. Invest. 97(9):2011-2019.
Reno et al. 1997. BioTechniques 22(6):1082-1086.
Rogachefsky et al., 1993. Osteoarthritis & Cartilage 1:105-114.
Rolling et al., 1996. FEBS lett. 393:53-56.
Rui et al., 1994. J. Biol. Chem. 269:5364.
Rutter et al. 1997. J. Cell. Biochem. 66:322-336.
Saijo et al., 1997. J. Exp. Med. 185:351-356.
Scharenberg et al., 1995. EMBO J. 14:3385-3395.
Schindler and Darnell Jr., 1995. Ann. Rev. Biochem. 64:621-651.
Schumacher et al., 1999. Prenat. Diagn. 19(7):653-656.
Seger and Krebs 1995. FASEB J 9:726-735.
Sharpe et al. WO 97/11692 (3 April 1997).
Silvennoinen et al., 1997. APMIS 105:497-509.
Stahl et al., 1994. Science 263:92.
Starr et al., 1997. Nature 387:917-921.
Stephenson, et al., 1987. Biochem. Biophys. Res. Comm. 144:583.
Stockwell, 1991. Clin. Anat. 4:161-191.
Subramaniam et al., 1999. Biochem. Biophys. Res. Commun. 262(1):14-19.
Sudbeck et al., 1999. Clin. Cancer Res. 5, 1569-1582.
Taniguchi, 1995. Science 268:251-255.
Thomis and Berg, 1997. J. Exp. Med. 185:197-206.
Thomis et al., 1995. Science 270:794-797.
Tortolani et al., 1995. J. Immunol. 155:5220-5226.
Tortorella et al., 1999. Science. 284: 1664-1666.
Uckun et al., 1999. Clin. Cancer Res. 5(10):2954-2962.
Urban, 1994. Br. J. Rheumatol 33: 901-908.
Van Beuningen et al., 1994. Lab. Invest. 71:279-290.
Velazquez et al., 1992. Cell 70:313.
Veldhuijzen et al., 1979. J. Cell. Physiol. 98: 299-306.
Venn et al., 1993. Arthritis Rheum. 36:819-826.
Villa et al., 1996. Blood 88:817-823.
Wang et al., 1999. J. Immunol. 162(7):3897-3904.
Watling et al., 1993. Nature 366:166.
Wilks, 1989. PNAS USA 86:1603.
Wilks et al., 1991. Mol. and Cell. Biol. 11:2057.
Wilson, 1988. In: Medicine For the Practicing Physician. Hurst et al. (eds.) 2d edition pp. 202-204. Witthuhn et al., 1993. Cell 74:227.
Witthuhn et al., 1994. Nature 370:153-157.
Witthuhn et al., 1999. Lymphoma Leukemian 32:289-297.
Yoshimura et al., 1995. EMBO J. 14:2816-2826.
Yu et al., 1998. J. Immunol. 161:218-227.
Zhang et al., 1996. PNAS USA 93:9148-9153.
Zuoning et al. 1999. Pharmacol. Exp. Therap. 291(1):124-130.

## Claims

1. The use of a compound which inhibits JAK-3, other than hymenialdisine (H) or debromohymenialdisine (DBH), in the manufacture of a pharmaceutical composition for inhibiting the progression or the likelihood of developing primary generalized osteoarthritis.

2. The use according to Claim 1, wherein the compound which inhibits JAK-3 is 4-(4'-hydroxyphenyl)-amino-6,7-dimethoxyquinazoline (WHI-P131).

## Patentansprüche

1. Verwendung einer Verbindung, die JAK-3 inhibiert, die nicht Hymenialdisin (H) oder Debromohymenialdisin (DBH) ist, zur Herstellung einer pharmazeutischen Zusammensetzung zur Inhibierung des Fortschreitens oder der Wahrscheinlichkeit, primäre generalisierte Osteoarthritis zu entwickeln.

2. Verwendung gemäß Anspruch 1, wobei die Verbindung, die JAK-3 inhibiert, 4-(4'-Hydroxyphenyl)-amino-6,7-dimethoxychinazolin (WHI-P131) ist.

## Revendications

1. Utilisation d'un composé inhibant la JAK-3, différent de l'hymenialdisine (H) ou de debromohymenialdisine (DBH), dans la fabrication d'une composition pharmaceutique pour inhiber l'évolution ou la probabilité de développement d'une ostéoarthrite primitive généralisée.

2. Utilisation selon la revendication 1, dans laquelle le composé inhibant la JAK-3 est 4-(4'-hydroxyphenyl)-amino-6,7-diméthoxyquinazoline (WHI-P131).
